# EUROPEAN PATENT SPECIFICATION

(11) **EP 4 393 529 B1**
(45) Date of publication and mention of the grant of the patent: **27.05.2026**
(21) Application number: 22307062.4
(22) Date of filing: 29.12.2022
(51) Int. Cl.: A61M 1/34, A61M 1/36

(54) **DISPOSABLE SET, APPARATUS AND PROCEDURE OF BLOOD RECIRCULATION IN AN EXTRACORPOREAL BLOOD TREATMENT APPARATUS**
EINWEGSET, VORRICHTUNG UND VERFAHREN ZUR BLUTREZIRKULATION IN EINER EXTRAKORPORALEN BLUTBEHANDLUNGSVORRICHTUNG
ENSEMBLE JETABLE, APPAREIL ET PROCÉDURE DE RECIRCULATION DU SANG DANS UN APPAREIL DE TRAITEMENT EXTRACORPOREL DU SANG

(43) Date of publication of application: 03.07.2024
(73) Proprietor: Gambro Lundia AB, 226 43 Lund (SE)
(72) Inventor: POUCHOULIN, Dominique, 01390 TRAMOYES (FR); FRUGIER, Alain, 69380 CHAZAY D'AZERGUES (FR)
(74) Representative: PGA S.p.A., Milano, Succursale di Lugano

(56) References cited:
- EP-B1- 1 590 017
- US-A1- 2015 292 529

## Description

### FIELD OF THE INVENTION

The present invention relates to a disposable set for an extracorporeal blood treatment apparatus configured to allow for fluid/blood recirculation in loop, for example when an alarm is issued stopping the blood pump, and the treatment has to be (e.g., temporarily) interrupted.

The invention also relates to an apparatus for extracorporeal blood treatment to perform fluid/blood recirculation in loop in a disposable set of an extracorporeal blood treatment apparatus.

The extracorporeal blood treatment apparatus of the present invention may perform dialysis treatments such as hemodialysis (HD) treatments, hemofiltration (HF) treatments, hemodiafiltration (HDF) treatments, or ultrafiltration (UF) treatments, as well as Continuous Renal Replacement Therapy (CRRT) treatments; the apparatus may also be configured for ECCO2R or oxygenation treatments, as well as for adsorption, hemoperfusion, liver treatments, or plasmapheresis.

### BACKGROUND

The kidneys fulfil many functions, including the removal of water, the excretion of catabolites (or waste from the metabolism, for example urea and creatinine), the regulation of the concentration of the electrolytes in the blood (e.g. sodium, potassium, magnesium, calcium, bicarbonates, phosphates, chlorides) and the regulation of the acid/base equilibrium within the body, which is obtained in particular by the removal of weak acids and by the production of ammonium salts. In individuals who have lost (temporarily or permanently) the use of their kidneys, since these excretion and regulation mechanisms no longer work, the body accumulates water and waste from the metabolism and exhibits an excess of electrolytes, as well as, in general, acidosis, the pH of the blood plasma shifting downwards, below 7.35 (the blood pH normally varies within narrow limits of between 7.35 and 7.45). As mentioned, in order to overcome renal dysfunction, resort is conventionally made to a blood treatment involving extracorporeal circulation through an exchanger having a semipermeable membrane (dialyzer) in which the patient's blood is circulated on one side of the membrane and a dialysis liquid, comprising the main electrolytes of the blood in concentrations close to those in the blood of a healthy subject, is circulated on the other side. Furthermore, a pressure difference is created between the two compartments of the dialyzer which are delimited by the semipermeable membrane, so that a fraction of the plasma fluid passes by ultrafiltration through the membrane into the compartment containing the dialysis liquid. The blood treatment which takes place in a dialyzer as regards waste from the metabolism and electrolytes results from two mechanisms of molecular transport through the membrane. On the one hand, the molecules migrate from the liquid where their concentration is higher to the liquid where their concentration is lower. This is diffusive transport. On the other hand, certain catabolites and certain electrolytes are entrained by the plasma fluid which filters through the membrane under the effect of the pressure difference created between the two compartments of the exchanger.

This is convective transport. Three of the abovementioned functions of the kidney, namely the removal of water, the excretion of catabolites and the regulation of the electrolytic concentration of the blood, are therefore performed in a conventional blood treatment device by the combination of dialysis and blood filtration (this combination is referred to as hemodiafiltration). To perform one or more of the above described treatments, the extracorporeal blood treatment equipment may comprise a disposable set including a plurality of lines for delivering fluid directly to the patient or into the extracorporeal blood circuit.

The disposable set for extracorporeal blood treatment commonly comprises a fluid circuit including fluid lines, blood lines and a filtration unit. The disposable set is usually engaged to a front panel of an extracorporeal blood treatment apparatus for delivering any one of the above mentioned treatments.

In case of extracorporeal blood circulation, one relevant concern includes reducing the risk of blood clotting within the blood lines of the disposable set.

However, during an extracorporeal blood treatment, it may be necessary to stop the blood pump (i.e., stop the extracorporeal blood circulation) when the extracorporeal blood is still in the blood circuit of the apparatus, for example, when an alarm is issued requiring to temporally suspend the treatment to allow the operator to fix the problem. In such a situation, the blood remains stationary in the blood lines increasing the risk of blood clotting both in the blood lines and in the filtration unit. Blood clotting risk increases with passing time, therefore, the operator should be able to resume the treatment in the shortest time possible. In such alarm situations with the blood pump stopped, stressful situations are caused on the operators, particularly the less experienced ones. In case blood clots in the lines and/or in the filtration unit, the disposable set has to be disposed and replaced with a new one. This leads to substantive loss of blood from the patient and determines an increase of the treatment costs and time.

Document EP1590017 discloses an extracorporeal blood treatment machine with a blood circuit including an inlet line leading to a filtration unit and an outlet line from the filtration unit. A fluid circuit comprises an inlet line leading to the filtration unit and an outlet line from the filtration unit so as to allow a fluid taken from a primary container to circulate within the filtration unit. An infusion line supplied by an auxiliary fluid container is provided for infusing in the outlet line of the blood circuit. The infusion line has an infusion branch injecting fluid in the inlet line of the blood circuit so as to enable the intensive therapy machine to manage therapies with large exchange of fluids both in pre- and post-infusion.

Document US20150292529 discloses a system for treating blood, which includes a single cassette capable of carrying out the various CRRT treatments. The circuit includes a pre-dilution line and a post-dilution line to deliver substitution fluid where the therapy requires. The cassette has one chamber on the blood withdrawal line in correspondence of the pre-infusion line and another chamber on the blood return line in correspondence of the post-infusion line. A channel connects the two chambers. In case of a problem a pump in the blood circuit continues to work and collects blood from the second chamber in order to circulate blood in a loop between the first and second chambers and the filter.

### AIM OF THE INVENTION

The aim of the invention is therefore to at least partially solve one or more of the drawbacks and/or limitations described above.

A first aim is to reduce the risk of clotting in the extracorporeal blood circuit by prevention of blood stagnation. A further object is to more easily address alarms that stop the blood pump and thus induce a risk of circuit clotting if troubleshooting is delayed, preventing, or at least reducing, the risk of blood clotting in the lines of the disposable set.

A further aim is to give the practitioner more time (thus also reducing stress) for troubleshooting, such as fixing treatment or patient issues and/or resetting apparatus parameters, during the temporary interruption in the treatment.

An additional aim is to improve the life time of the disposable set, which is particularly relevant in CRRT therapies.

A further aim is to avoid the blood volume housed in the disposable set to be discarded, which results in blood loss for the patient and the disposal of the circuit, thus also reducing waste during extracorporeal blood treatments.

An auxiliary aim is to allow for recirculation in the blood withdrawal line to the maximum extent possible so to avoid blood stagnation in a long portion of the blood lines.

These objects and more, which will appear more from the following description, are substantially achieved by a disposable set, and an apparatus, in accordance with one or more of the following claims.

Not all aims may be achieved by each and every embodiment of the invention.

### SUMMARY SECTION

The disposable set of the invention is a disposable set (100) for an extracorporeal blood treatment apparatus (1) comprising:
- a filtration unit (2) having a blood inlet (2a) and a blood outlet (2b);
- a blood circuit (17) including:
   > a blood withdrawal line (6) extending between a first end (6a) connected to the blood inlet (2a) of the filtration unit (2), and a second end (6b) for direct or indirect connection to a patient (P);
   > a blood return line (7) extending between a first end (7a) connected to the blood outlet (2b) of the filtration unit (2), and a second end (7b) for direct or indirect connection to the patient;
   > a blood pump tract (6p) located on the blood circuit (17) and configured to be engaged to a blood pump (21) of the extracorporeal blood treatment apparatus to promote blood flow in the blood circuit,
- an infusion line (63) extending from a first end (63a) for connection to an infusion fluid source (64),
- a pre-blood-pump infusion line (51) extending from a first end (51a) for connection to a second fluid source (10), and a second end (51b) fluidly connected to the blood withdrawal line (6) between the second end (6b) of the blood withdrawal line (6) and the blood pump tract (6p), wherein the infusion line (63) branches off at a branch site (67) towards:
   > a second end (63b) of the infusion line (63); and
   > a third end (63c) of the infusion line (63) in direct or indirect fluid communication with the blood return line (7) at a second site (32), the second site (32) being interposed between the blood pump tract (6p) and the second end of the blood return line (7);

   characterized in that the second end (63b) of the infusion line (63) is directly connected to the pre-blood-pump infusion line (51) and in fluid communication with the blood withdrawal line (6) at the first site (31), the first site (31) being interposed between the second end (6b) of the blood withdrawal line (6) and the blood pump tract (6p);
   wherein the infusion line (63) and the pre-blood-pump infusion line (51) are connected together and present a common infusion tract extending up to the first site (31) of the blood withdrawal line (6), wherein the blood circuit is designed to allow fluid flow in a loop recirculation circuit (11) including:
- a portion of the blood withdrawal line (6) interposed between the first site (31) of the blood withdrawal line (6) and the blood inlet (2a) of the filtration unit (2);
- the blood pump tract (6p);
- the filtration unit (2);
- a portion of the blood return line (7) interposed between the blood outlet (2b) of the filtration unit (2) and the second site (32) of the blood return line (7);
- a recirculation portion (66) of the infusion line (63) downstream the branch site (67) and interposed between the first site (31) and the second site (32);
- the common infusion tract;
the loop recirculation circuit (11) being configured to allow blood or a fluid, at least in a recirculation condition, to continuously flow in a loop circulation.

The apparatus of the invention is an extracorporeal blood treatment apparatus including:
- one or more pumps (21, 25, 26, 54, 65, 75) comprising a blood pump (21),
- one or more intercepting elements (20, 27, 59, 70),
- a disposable set (100) in accordance with any one of the previous claims
- a control unit (12) configured to control said one or more pumps (21, 25, 26, 54, 65, 75) and said one or more intercepting elements (20, 27, 59, 70) in order to define different flow circuits inside the blood circuit (17) and the infusion line (63), the control unit (12) being configured to define at least the following flow circuits:
   > a treatment flow circuit, wherein the one or more pumps (21, 25, 26, 54, 65, 75) and the one or more intercepting elements (20, 27, 59, 70) are configured to allow blood to flow from the second end (6b) of the blood withdrawal line (6) to the filtration unit (2) and from the filtration unit (2) to the second end (7b) of the blood return line (7); and
   > a loop recirculation circuit (11), wherein the one or more pumps (21, 25, 26, 54, 65, 75) and the one or more intercepting elements (20, 27, 59, 70) are configured to allow blood or a fluid to continuously flow in a loop circulation,
wherein the infusion line (63) and the pre-blood-pump infusion line (51) are connected together and present a common infusion tract extending up to the first site (31) of the blood withdrawal line (6), wherein the loop recirculation circuit (11) includes:
· a portion of the blood withdrawal line (6) interposed between the first site (31) of the blood withdrawal line (6) and the blood inlet (2a) of the filtration unit (2);
· the blood pump tract (6p);
· the filtration unit (2);
· a portion of the blood return line (7) interposed between the blood outlet (2b) of the filtration unit (2) and the second site (32) of the blood return line (7);
· a recirculation portion (66) of the infusion line (63) downstream the branch site (67) and interposed between the first site (31) and the second site (32);
· the common infusion tract.

### DRAWINGS

Some embodiments and some aspects of the invention will be described below with reference to the attached drawings, provided for illustrative purposes only, wherein:
- Figure 1 is a schematic view of the fluid lines of a disposable set coupled to pumps, sensors and actuators of a related apparatus according to an embodiment not part of the present invention:
- Figure 1a represents the scheme of figure 1 with a recirculation loop highlighted by a thicker line;
- Figure 2 is a schematic view of the fluid lines of a disposable set coupled to pumps, sensors and actuators of a related apparatus according to a second embodiment of the present invention;
- Figure 2a represents the scheme of figure 2 with the recirculation loop highlighted by a thicker line;
- Figure 3 is a representation of a disposable set coupled to pumps, sensors and actuators of a related apparatus according to a third embodiment of the present invention;
- Figure 3a mirrors the scheme of figure 3, wherein the recirculation loop is schematically highlighted by a thicker line;
- Figure 4 is a representation of a further embodiment of a disposable set mounted on a related treatment apparatus according to a fourth embodiment not part of the present invention;
- Figure 4a mirrors the scheme of figure 4, wherein the recirculation loop is schematically highlighted by a thicker line;
- Figure 5 is a representation of an alternative embodiment of a disposable set mounted on a related treatment apparatus according to a fifth embodiment not part of the present invention;
- Figure 5a mirrors the scheme of figure 5, wherein the recirculation loop is schematically highlighted by a thicker line.

### DEFINITIONS

In this detailed description, corresponding parts illustrated in the various figures are indicated with the same numerical references. The figures may illustrate the invention by means of non-scale representations; therefore, parts and components illustrated in the figures relating to the object of the invention may relate exclusively to schematic representations.

### Upstream and downstream

The terms upstream and downstream refer to a direction or trajectory of advancement of the fluid or blood configured to flow within a line of the disposable set during a usual blood treatment procedure, wherein blood is withdrawn from the patient at a first access site, circulated into the blood withdrawal line, the filtration unit and the blood return line before being re-injected into the patient at a second access site. Infusion lines directs the respective fluid into the extracorporeal blood circuit and the fresh dialysis fluid is fed to the filtration unit (countercurrent to the blood) and removed via an effluent line towards a drain.

### DETAILED DESCRIPTION

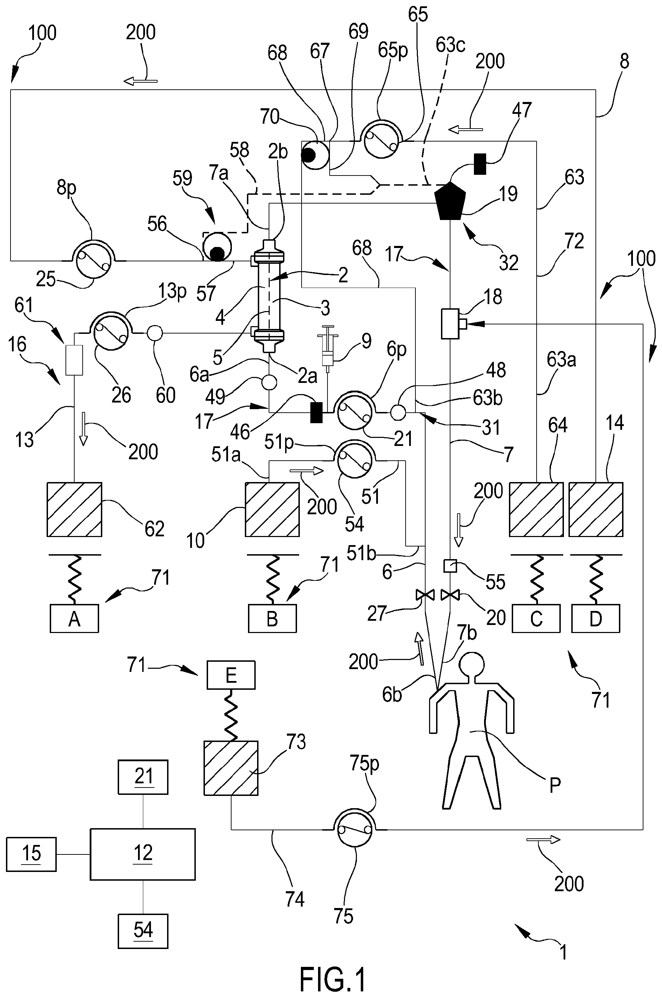

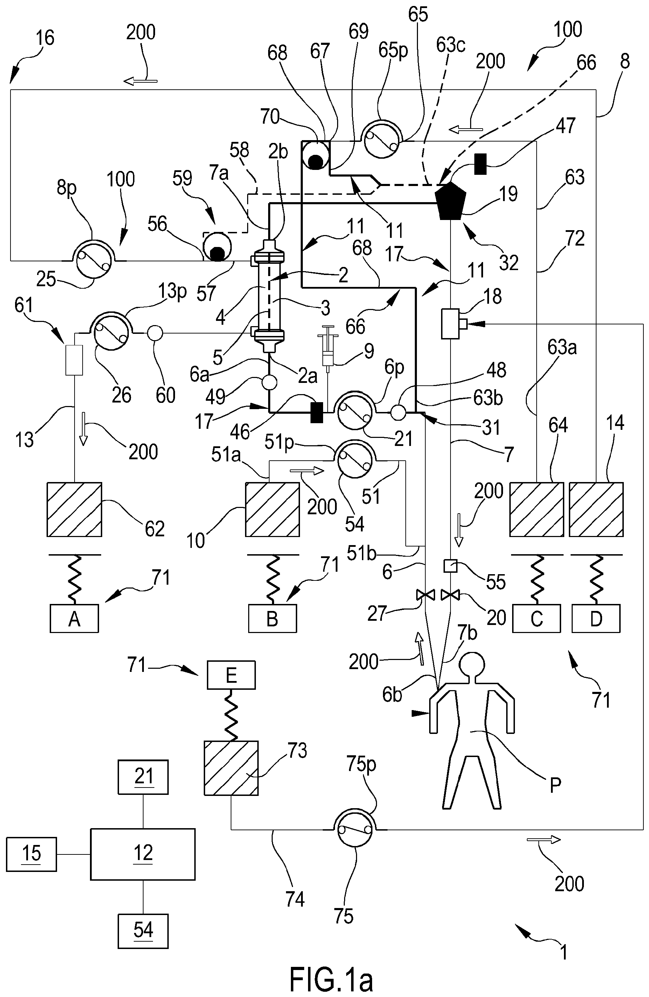

With reference to figure 1, reference number 1 generally refers to the extracorporeal blood treatment apparatus, particularly, but not exclusively, for intensive care therapies. The extracorporeal blood treatment apparatus of the present invention may perform any dialysis treatments such as hemodialysis (HD), hemofiltration (HF), hemodiafiltration (HDF), or ultrafiltration (UF), as well as (C)RRT treatments; the apparatus may alternatively/additionally be configured for ECCO2R or oxygenation treatments, as well as for adsorption, liver treatments, hemoperfusion, or plasmapheresis. The extracorporeal blood treatment apparatus 1 is designed to receive a disposable set 100 as claimed in the present application and below described.

The apparatus according to figure 1 is particularly (but not exclusively) designed for Renal Replacement Therapies (RRT) and particularly for Continuous Renal Replacement Therapies (CRRT). CRRT apparatuses are configured to deliver very specific treatments designed for patients versing in acute states of illness and who have temporarily lost their kidney function. In this respect, CRRT systems may be structurally and/or operationally different from extracorporeal blood treatment systems designed for chronic patient care. CRRT systems need to exhibit specific technical features enabling the system to:
- allow setting of a weight loss rate,
- continuously remove excess water in accordance with a set weight loss rate,
- operate continuously at comparably low flow rates compatible with CRRT, and
- balance ion equilibrium by means of proper dialysis being performed and/or by means of substitution fluid continuously being delivered at controlled flow rates.

Finally, in order to prepare a CRRT apparatus as soon as possible given the acute situation of a patient requiring a treatment and given the absence of advance notice of the emergency situation requiring treatment, the CRRT machine is dressed using an integrated disposable set 100, wherein all the lines and the filtration unit are grouped together and already properly connected in the disposable set. Further, all the fluids are normally (but not necessarily) contained in pre-packaged bags (dialysis fluid or replacement fluids in bags of e.g., 2, 5 or 10 litres each) or pre-packaged syringes (heparin and/or concentrated calcium replacement solution).

Notwithstanding the specifically described embodiments are referred to extracorporeal blood treatment apparatus and disposable sets for acute therapy, the present invention is not limited to such field and may also be implemented and used in apparatus for the treatment of chronic patients.

Reference number 100 is directed to a disposable set for the extracorporeal blood treatment apparatus. The disposable set 100, schematically shown in figures 1-5, comprises blood lines and fluid lines respectively configured to be engaged to the treatment apparatus 1, the latter comprising for example peristaltic pumps to promote fluid and blood flows.

The disposable set 100 comprises at least one filtration unit 2, which is configured to treat the blood withdrawn from the patient. The filtration unit 2 may be any kind of filter to treat blood, such as a filter for performing haemodialysis (HD), ultrafiltration (UF), haemofiltration (HF) and haemodiafiltration (HDF), for hemoperfusion, a plasmafilter, an adsorber, etc...

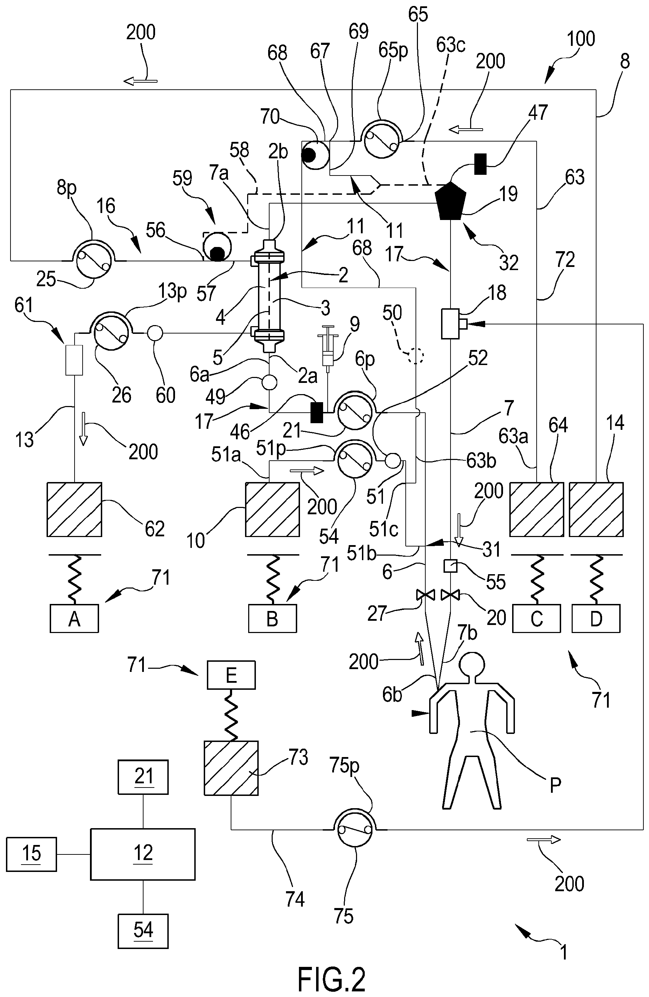

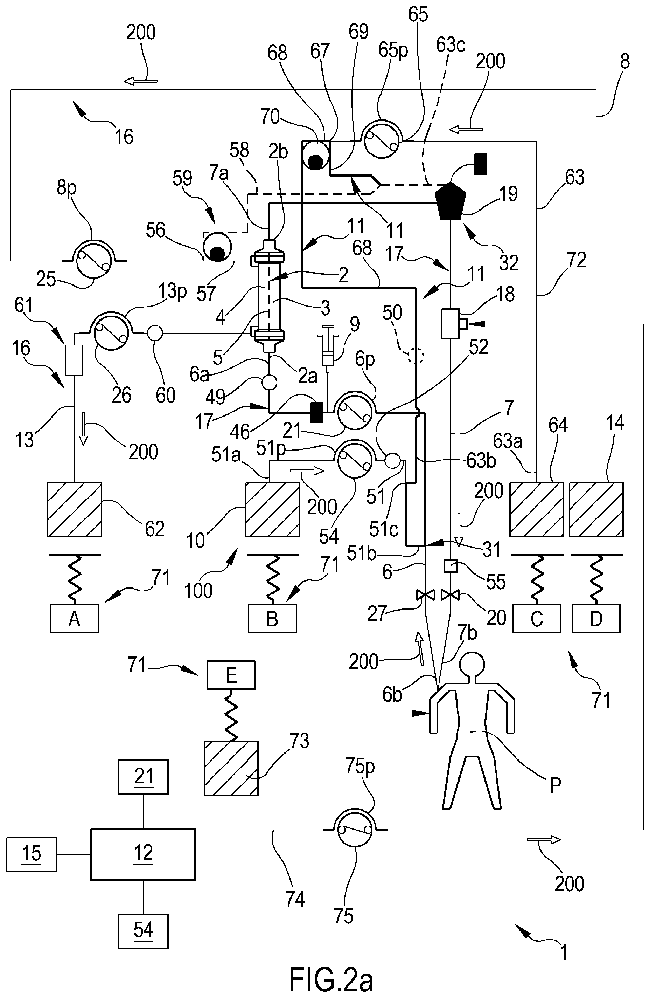

As shown in figures 1 and 2, in certain embodiments, the filtration unit 2 has a primary chamber 3 and a secondary chamber 4 separated by a semi-permeable membrane 5, wherein the primary chamber 3 receives blood withdrawn from the patient, while the secondary chamber 4 receives waste products filtered out from the blood and discharges them through a fluid outlet connected to an effluent fluid line 13. Depending upon the treatment, the membrane 5 of the filtration unit 2 may be selected to have different properties and performances. A blood circuit 17 comprises a blood withdrawal line 6 extending between a first end 6a connected to a blood inlet 2a of the primary chamber 3 of the filtration unit 2, and a second end 6b for connection to a patient P. The blood withdrawal line 6 is configured to receive blood from the patient P and to allow blood circulation along a withdrawal direction 200 from the second end 6b towards the first end 6a of the blood withdrawal line 6 and into the filtration unit 2.

The blood circuit 17 further comprises a blood return line 7 extending between a first end 7a connected to a blood outlet 2b of the primary chamber 3 of the filtration unit 2, and a second end 7b for connection to the patient P. The blood return line 7 is configured to receive (treated) blood from the blood outlet 2b of the filtration unit 2 and to allow blood circulation along a return direction from the first end 7a towards the second end 7b of the blood return line 7.

The blood withdrawal line 6 and the blood return line 7 are usually made of a flexible material, for example PVC or a plastic based material, and may also be transparent to allow an operator to see the blood flowing within the lines.

The blood circuit 17 further comprises a pump tract 6p configured to engage a blood pump 21 of the extracorporeal blood apparatus to promote blood flow in the blood withdrawal line 6 and in the blood return line 7. The pump tract 6p may be arranged on the blood withdrawal line 6 or on the blood return line 7. Alternatively, the blood circuit 17 may comprise two blood pumps, wherein a first blood pump is engaged to the blood withdrawal line 6, and a second blood pump is engaged to the blood return line 7 (this embodiment is not shown).

Please note that pump symbols in the drawings do not necessarily indicate the direction of the flow inside the respective pump tract. Generally, each and every (e.g., peristaltic) pump may be controlled in one direction and in the opposite direction, too, depending on the apparatus and therapy needs.

As shown in the embodiments of figures 1 to 5, the blood withdrawal line 6 includes the pump tract 6p that is engaged by the blood pump 21 of the extracorporeal blood treatment apparatus. The blood pump 21 is configured to determine a blood flow in a direction 200 from the second end 6b of the withdrawal line towards the filtration unit 2 and then to back to the blood return line 7.

In a first example and as represented in the drawings, the blood pump tract 6p may be a portion of the blood withdrawal line 6 itself, which is interposed between the first end 6a and the second end 6b of the blood withdrawal line 6. The blood pump tract 6p may be different with respect to the other line tracts of the blood withdrawal line 6 in terms of dimension (e.g. passage section and/or wall thickness) and/or material. In particular the blood pump tract 6p may have an external dimension, i.e. an external diameter, larger than an external dimension, i.e. an external diameter, of the first and/or second tracts of the blood withdrawal line 6. In addition or alternatively, the blood pump tract 6p may have an internal dimension, i.e. an inner diameter, defining the fluid passage of the blood pump tract 6p, which is larger than an internal dimension, i.e. an internal diameter, of the first and/or second tracts of the blood withdrawal line. The blood pump tract 6p may be a separate and different tube segment with respect to the other line tracts of the blood withdrawal line 6, wherein the blood pump tract 6p is engaged/coupled to the other tracts by gluing or welding process during a manufacturing process of the disposable set 100 (and/or a suitable connector may be interposed between the end of the pump tract and the other tube portions of the withdrawal line to join the respective ends).

The blood pump tract 6p may also be curved (e.g., a Ω shape) and/or may have elastic properties different from the properties of the first and the other line tracts of the blood withdrawal line 6: for example the blood pump tract 6p may be more flexible than the other line tracts of the blood withdrawal line 6 e.g., to withstand the fatigue stresses caused by the peristaltic pump 21 of the blood treatment apparatus.

In an alternative, the blood pump tract 6p may be a disposable pump header to be connected to the pump body e.g., by magnetic coupling. The blood pump 21 may be a centrifuge pump. This is for example the case of a centrifuge pump that may be used in ECMO circuit

During treatment, the blood withdrawal line 6 and the blood return line 7 are connected directly or indirectly to the blood cardiovascular system of the patient through an access device such as a needle, a catheter, or a graft. Normally, in a renal replacement treatment the blood withdrawal line 6 and the blood return line 7 are directly connected to the blood cardiovascular system of the patient; however, in certain situations, such as when the patient is subject to a cardiopulmonary by-pass and therefore already connected to an ECMO machine, the blood withdrawal line 6 and the blood return line 7 may be connected to the ECMO extracorporeal blood lines and consequently indirectly connected to the blood cardiovascular system of the patient (via the ECMO blood lines).

The apparatus 1 comprises a blood pump 21 for controlling the blood flow in the blood circuit 17. The blood pump 21 is generally a peristaltic pump acting either on the blood withdrawal line 6 (as shown e.g. in figures 1-5) and/or on the blood return line 7 (embodiment not shown). An operator may enter a set value for the blood flow rate through a user interface 15 and a control unit 12, during treatment, is configured to control the blood pump based on the set blood flow rate.

Blood is withdrawn from the patient at a vascular access and flows through the blood withdrawal line 6; after passing through the primary chamber 3 of the filtration unit 2, where the suitable exchanges of substances, molecules and fluids occur through the semipermeable membrane 5, the treated blood enters the blood return line 7, first passing through the air separator, commonly known as "*bubble trap*", 19 designed to ensure removal of air bubbles present in the blood. The treated blood getting out of the bubble trap 19, before being returned to the patient P passes through an air bubble sensor 55 verifying the absence of air bubbles within the treated blood that has to be re-introduced in the patient's blood vascular circuit at the venous access. Downstream the air bubble sensor 55, a safety valve 20 (or venous clamp) is provided: the safety valve 20, in case of alarm requiring stop of the blood pump and patient isolation, blocks the blood flow towards the patient. For example, should the bubble sensor 55 detect the presence of air/gas in the blood flow, the control unit 12 controls the safety valve 20 to the close position to prevent air to be infused into the patient.

A corresponding safety valve 27 (or arterial clamp) may be present on the blood withdrawal line 6 close to the patient vascular access to fully isolate the patient from the extracorporeal blood circuit in case of need.

The safety valve 27 of the blood withdrawal line 6 and the safety valve 20 of the blood return line 7 may be arranged both in the close position so that the patient P is fluidly isolated from the blood circuit 17 of the disposable set. The safety valve 27 of the blood withdrawal line 6 and the safety valve 20 of the blood return line 7 may be manually controllable, but are usually automatically controllable by the control unit 12 between the close position and the open position, and vice versa.

The disposable set 100 further comprises an infusion line 63 extending from a first end 63a, for connection to an infusion fluid source 64, and a branch site 67 branching off the infusion line 63 towards a second end 63b and towards a third end 63c. The portion of the infusion line 63 interposed between the second end 63b and the third end 63c of the infusion line 63 defines a recirculation portion 66 of the infusion line, as described in more detail here-after in the present description.

The fluid source 64 is a source of infusion or replacement fluid. In particular, the fluid source 64 may be a plastic bag housing a pre-packaged replacement fluid solution.

The second end 63b of the infusion line 63 is in direct or indirect fluid communication with the blood withdrawal line 6 at a first site 31. A direct fluid communication between the infusion line 63 and the blood withdrawal line 6 means that the infusion line 63 is directly connected to the blood withdrawal line 6 and the fluid exiting the second end 63b of the infusion line 63 enters directly into the blood stream flowing in the blood withdrawal line 6 of the blood circuit 17.

An indirect fluid communication between the infusion line 63 and the blood withdrawal line 6 means that the infusion line 63 is not directly connected to the blood withdrawal line 6, but to another fluid line (e.g., PBP line 51) that is itself directly connected to the blood withdrawal line 6. As a consequence, the infusion fluid exiting the second end 63b of the infusion line 63 enters the other fluid line and then reaches the blood withdrawal line 6 at the injection point of the other line into the blood withdrawal line 6.

As below further explained, an example of direct fluid communication is shown in figures 1 and 1a (and 4, 4a, 5, 5a), while an example of indirect fluid communication is shown in figures 2 and 2a (and 3, 3a).

The first site 31 is located on the blood circuit 17 and is specifically arranged on the blood withdrawal line 6 and is interposed between the blood pump tract 6p and the second end 6b of the blood withdrawal line 6. Even though not essential, the first site 31 may be arranged as close as possible to the patient, so that the remaining portion of the withdrawal line 6 between the first site 31 and the patient is as short as possible. The first site 31 is upstream the blood pump tract 6p with respect to the blood withdrawal direction 200.

As apparent, the first site 31 may or may not coincide with the second end 63b of the infusion line 63. In some embodiments, the first site 31 is coincident with the location where the second end 63b of the infusion line 63 (directly) connects to the blood withdrawal line 6 (e.g., fig. 1); in other embodiments, the first site 31 (which is on the blood withdrawal line 6) may be distinct and spatially separated from the second end 63b of the infusion line 63 (e.g., fig. 2).

The first site 31 may comprise a fluid connector, i.e. a three-way fluid connector, including a blood inlet (directly) fluidly connected to the withdrawal line 6 and facing the second end 6b of the blood withdrawal line 6, a blood outlet (directly) fluidly connected to the withdrawal line 6 and facing the first end 6a of the blood withdrawal line 6, in particular facing the pump tract 6p of the blood withdrawal line 6, and an infusion inlet (directly or indirectly) in fluid communication with the second end 63b of the infusion line 63.

The fluid connector may be made by plastic material stiffer than the material of the infusion line 63 and/or of the blood withdrawal line 6. On the contrary, the infusion line 63 is flexible, in particular more flexible than the fluid connector. Analogously, the blood withdrawal line 6 is more flexible than the fluid connector.

Notably, the first site 31 is distinct and spatially separated from the second end 6b of the blood withdrawal line 6.

The third 63c end of the infusion line 63 is in direct or indirect fluid communication with the blood return line 7 at a second site 32.

The second site 32 is interposed between the blood pump tract 6p and the second end 7b of the blood return line 7. In particular, when the blood pump tract 6p is on the blood withdrawal line 6, the second site 32 is interposed between the blood outlet 2b of the filtration unit 2 and the second end 7b of the return line 7.

The second site 32 may comprise a fluid connector, too, i.e. a three-way fluid connector, including a blood inlet (directly) fluidly connected to the return line 7 and facing the first end 7a of the blood return line 7, a blood outlet (directly) fluidly connected to the return line 7 and facing the second end 7b of the blood return line 7, and an infusion inlet (directly or indirectly) in fluid communication with the third end 63c of the infusion line 63.

According to the embodiment shown in figures 1 and 2, a bubble trap 19 may be located at the second site 32 arranged on the blood return line 7. The bubble trap 19 includes a blood inlet (directly) fluidly connected to the return line 7 and facing the first end 7a of the blood return line 7, a blood outlet (directly) fluidly connected to the return line 7 and facing the second end 7b of the blood return line 7, and an infusion inlet (directly) fluidly connected to the third end 63c of the infusion line 63. The bubble trap 19 is configured to separate air/gas bubbles from the blood stream, in order to prevent bubbles to flow towards the patient.

Notably, the second site 32 is distinct and spatially separated from the second end 7b of the blood return line 7.

As previously mentioned, the infusion line 63 extends from the first end 63a thereof for a first tract 72 up to the branch site 67, where the first tract 72 branches into a second tract 68, extending from the branch site 67 up to the second end 63b of the infusion line 63, and into a third tract 69 extending from the branch site 67 up to the third end 63c of the infusion line 63. A recirculation portion 66 of the infusion line 63 comprises (and may be entirely defined by - in the case of figures 1, 1a, 4, 4a, 5, and 5a) the second tract 68 and the third tract 69 of the infusion line 63.

The infusion line 63 further comprises a respective infusion pump tract 65p, interposed between the first end 63a and the branch site 67 of the infusion line 63; the infusion pump tract 65p of the infusion line 63 is configured to be engaged by a respective infusion pump 65 of the extracorporeal blood treatment apparatus, i.e. an occlusive pump such as a peristaltic pump, configured to cause a fluid flow in a direction from the first end 63a of the infusion line towards the branch site 67. The infusion pump tract 65p may comprise the same technical features previously described and differentiating the blood pump tract 6p from the other line tracts of the blood withdrawal line 6.

Correspondingly, the apparatus 1 includes the infusion pump 65 (in the example a peristaltic pump) engaged to the infusion line 63 and configured to determine, during infusion, the flow rate along the line of the substance housed in the infusion fluid source 64, i.e. replacement fluid.

The extracorporeal blood treatment apparatus 1 may further comprise a selector 70 active on the infusion line 63 at the branch site 67. The selector 70 may be switchable (at least) between:
- a first operating position (pre-infusion), wherein the selector 70 allows fluid communication between the first tract 72 and the second tract 68 of the infusion line 63, and prevents fluid communication between the first tract 72 and the third tract 69 of the infusion line 63;
- a second operating position (post-infusion), wherein the selector 70 prevents fluid communication between the first tract 72 and the second tract 68 of the infusion line 63, and allows fluid communication between the first tract and the third tract 69 of the infusion line 63; and
- a recirculation position (neutral position), wherein the second tract 68 and the third tract 69 of the infusion line 63 are in fluidly communication with each other.

In particular, when the selector 70 is in the recirculation position, both the second tract 68 and the third tract 69 of the infusion line 63 define the recirculation portion 66 of the loop recirculation circuit 11. Furthermore, when the selector 70 is in the recirculation position, the first site 31 is in fluid communication with the second site 32 through the recirculation portion 66. In other terms, in the recirculation position, the selector 70 (which may be a valve clamping either the second tract 68 or the third tract 69 or none of the tubes 68, 69) is not acting on (clamping) any tube portions and is in a neutral and distanced position from the second and third tracts 68, 69. In the recirculation position, no fluid flows along the first tract 72 of the infusion line 63. For example, the infusion pump 65 may be an occlusive pump and may be inactive thereby preventing any flow; alternatively, a clamp, the same selector 70, or another device may prevent flow in the first tract 72 e.g., by closing it.

Differently, in either the first or the second operating position, fluid flows from the first tract 72 towards the second and/or third tracts 68, 69 and infuse replacement fluid in the blood circuit. In this situation, the cooperation between an active infusion pump 65 and the selector 70 may define the quantity of fluid pre-infused and the quantity of fluid simultaneously post-infused.

Of course, in case only pre- or post-infusion at a time are manageable (i.e., either 100% in pre-infusion or 100% in post- infusion), then the selector 70 prevents fluid communication between the first site 31 and the second site 32 through the recirculation portion 66 of the loop recirculation circuit 11. In particular, in the first operating position, the passage of fluid from the fluid source 64 to the second tract 68 is allowed, while the passage of fluid from the fluid source 64 to the third tract 69 is prevented. On the contrary, in the second operating position, the passage of fluid from the fluid source 64 to the third tract 69 is allowed, while the passage of fluid from the fluid source 64 to the second tract 68 is prevented. Notably, the first operating position and the second operating position may be taken during a standard dialysis treatment to either pre- or post-infuse a replacement fluid into the extracorporeal blood circuit, while the recirculation position of selector 70 is taken to allow fluid recirculation in the recirculation condition.

Selector 70 may consist of a valve element operating on the infusion line 63 by alternatively blocking the passage of fluid in either branch and further placeable in the neutral position. Suitable selectors may be alternatively provided, which are able to establish a priori the amount of liquid that has to pass through both branches simultaneously. It will also be possible to vary the percentages of fluid in either branch as a function of time and of the pre-established therapies. Notably the selector 70 is connected to the control unit 12, wherein the latter is configured to control selectively the selector in said first operating position, in the second operating position and in the recirculation position.

The disposable set may further comprise a pre-blood-pump (PBP) infusion line 51 extending from a first end 51a for connection to a second fluid source 10, and a second end 51b fluidly connected to the blood withdrawal line 6. The pre-blood-pump infusion line 51 may be an anticoagulant infusion line 51; in such a case the second fluid source 10 may house a fluid solution including citrate and/or citric acid. The second fluid source 10 may be a plastic bag housing the PBP infusion fluid.

The second end 51b of the pre-blood-pump infusion line 51 is interposed between the second end 6b of the blood withdrawal line 6 and the blood pump tract 6p as shown in figures 1 and 2.

Further, the second end 51b of the pre-blood-pump infusion line 51 may be interposed between the second end 6b of the blood withdrawal line 6 and the first site 31 as shown in figure 1 in case of direct connection of the infusion line 63 to the blood withdrawal line 6. Indeed, in such a case, connection between second end 63b of the infusion line 63 and the withdrawal line is optionally downstream the second end 51b of the pre-blood-pump infusion line 51.

The pre-blood-pump infusion line 51 also comprises a respective PBP (pre-blood-pump) infusion pump tract 51p configured to be engaged by a respective PBP infusion pump 54 configured to infuse the substance from the second fluid source 10 through the pre-blood-pump infusion line 51 and into the blood circuit 17.

The PBP infusion pump 54 is operatively connected to the control unit 12 which is configured to selectively control the PBP infusion pump 54 to promote the infusion fluid to flow within the infusion line 51 and to deliver the infusion fluid into the blood withdrawal line 6: the control unit 12 may be configured to control the PBP infusion pump 54 to set the prescription flow rate. Also in this regard, the type of treatment, the infusion bag content, and other conditions determine the set infusion rate. The control unit may also be configured to stop the PBP infusion pump 54 to arrest delivery of the infusion fluid.

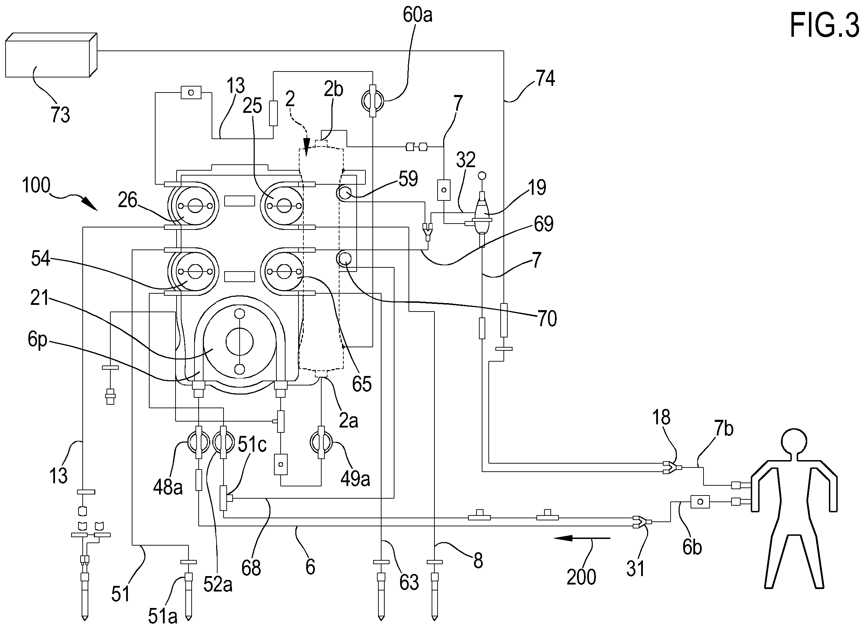

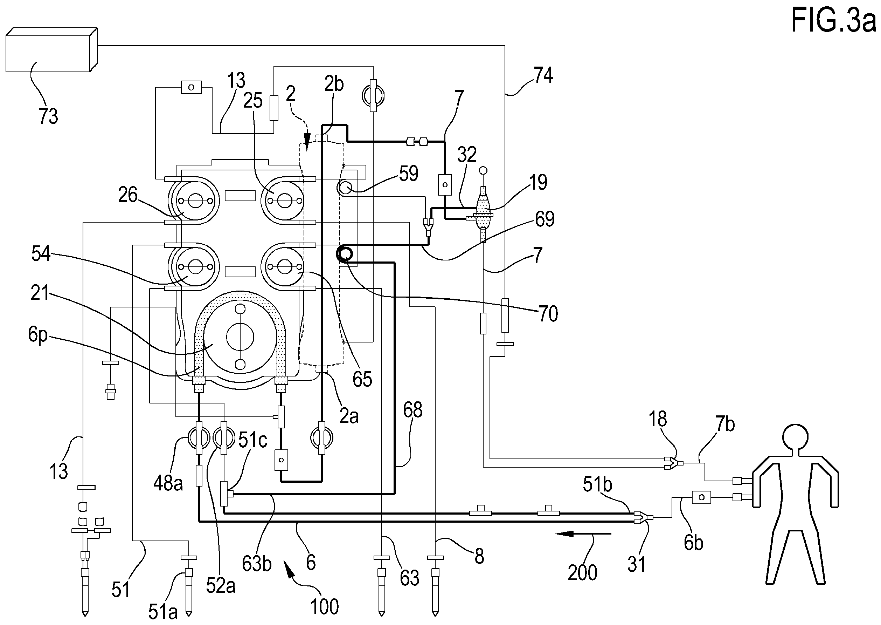

According to the embodiment of figures 2 and 3, the second end 51b of the pre-blood-pump infusion line 51 is connected to the blood withdrawal line 6 at the first site 31. In this case, the second tract 68 of the infusion line 63 is directly connected to the pre-blood-pump infusion line 51 at an access point 51c interposed between the second end 51b of the pre-blood-pump infusion line 51 and the PBP infusion pump tract 51p of the pre-blood-pump infusion line 51. In other terms, the infusion line 63 and the pre-blood-pump infusion line 51 are connected together, at the access point 51c, and fluid moves towards the blood withdrawal line 6 along a common infusion tract extending up to the first site 31 (i.e., the common infusion tract runs from the access point 51c to the second end 51b of the pre-blood-pump infusion line 51).

The disposable set further comprises an ion replacement fluid line 74 connected, on one end, to an ion replacement container 73 and, to the other end, to the blood return line (7), for example at an access site 18, such as a connector allowing infusion and homogenous mixing of the infused fluid with the blood. As shown in the enclosed figures, the access site 18 is placed downstream the second site 32 and possibly, but not necessarily, upstream the air bubble sensor 55. An ion replacement pump 75 acting on a ion replacement pump tract 75p allow to pump the fluid through the ion replacement infusion line 74.

In an alternative embodiment (not shown) the ion replacement infusion line 74 is directly connected to an additional patient vascular access directly infusing the ion replacement fluid into the patient blood (e.g., into a venous peripheral vessel of the patient).

Usually, the ion replacement infusion line 74 is used to infuse a calcium (and possibly a magnesium, too) concentrated solution to re-establish the proper electrolyte balance in the patient blood during treatments with regional anticoagulation using citrate.

According to the disclosed embodiments, the disposable set 100 further comprises a fluid supply line 8. The fluid supply line 8 delivers dialysis fluid from a dialysis liquid source 14 to the filtration unit 2. The dialysis liquid source 14 consists of one or more bags containing a suitable dialysis liquid designed to supply the supply line 8 of the dialysis fluid circuit 16 with dialysis fluid.

The dialysis liquid supply line 8 also comprises a respective supply pump tract 8p configured to be engaged by a dialysis fluid pump 25 of the extracorporeal blood treatment apparatus to promote dialysis fluid flow towards the fluid inlet of the secondary chamber 4 of the filtration unit.

The dialysis fluid pump 25, i.e. a peristaltic pump, defines a direction 200 of dialysis fluid circulation and controls the flow rate of the fresh dialysis liquid from the bag 64 towards the filtration unit 2.

In the embodiments of the figures, downstream from the dialysis fluid pump 25 in the direction of circulation 200 a branching 56 is provided splitting the dialysis supply line 8 up into an intake branch 57 and a post infusion tract 58. In other terms, the supply line 8 splits in a post infusion tract 58 extending up to the return line 7 so that the fluid supply line 8 is fluidly connected to the return line 7 of the blood circuit 17.

By means of said infusion branch 58 it is possible to obtain a post-infusion directly in the blood line 17 using the content of the primary fluid container 64.

The post infusion tract 58 may share an end portion with the third tract 69 of the infusion line 63, so that both infusion line 63 and the post infusion tract 58 are connected together and deliver fluid to the second site 32. In particular, the post infusion line 63 and the post infusion tract 58 may be connected to the bubble trap 19 of the disposable set. The shared end portion is interposed between the point wherein the infusion line 63 joins the post infusion tract 58 and the second site 32/the bubble trap 19.

In a different embodiment (not shown), the post infusion tract 58 may extend directly to the bubble trap 19 or to the blood return line 7, without sharing any portion with the infusion line 63.

Conversely, the intake branch 57 conveys the fluid directly to the filtration unit 2 and in particular to the secondary chamber 4 of said filtration unit. The dialysis fluid circuit 16 is further equipped with a selector 59 for determining the percentages of fluid flow within the infusion branch 58 and the intake branch 57. Generally said selector 59, usually placed near the branching 56, can be positioned at least between a first operating position in which it allows the passage of fluid in the intake branch 57 and blocks the passage in the post infusion line 58, and a second operating position in which it allows the passage of fluid in the post infusion line 58 and blocks the passage in the intake branch 57. In other words, said selector 59 may consist of a valve element operating on the dialysis fluid circuit 16 by alternatively blocking the passage of fluid in either branch. Suitable selectors may be alternatively provided, which are able to establish a priori the amount of liquid that has to pass through both branches simultaneously. It will also be possible to vary the percentages of fluid in either branch as a function of time and of the pre-established therapies. Notably the selector 59 may be connected to the control unit 12, wherein the latter is configured to control selectively the selector in said first operating condition and in the second operating condition.

The dialysis liquid through the intake branch 57 gets into the secondary chamber 4 of the filtration unit 2. In particular, the primary chamber 3 through which the blood flow passes is separated from the secondary chamber 4 through which the dialysis liquid passes by means of the semipermeable membrane 5 ensuring the suitable passage of the substances/molecules and of fluid from the blood towards the dialysis liquid by means of convection and diffusion processes, and further ensuring through diffusion the passage of substances/molecules from the dialysis liquid towards the blood.

The disposable set 100 further comprises an effluent fluid line 13 connected to the fluid outlet of the filtration unit 2: the effluent fluid line 13 may also comprise a respective effluent pump tract 13p configured to be engaged by an effluent pump 26. The fluid to be removed then passes through an effluent pressure sensor 60 and a blood detector 61 and is conveyed into a drain, such as a collection container or bag 62.

An actuator is provided for conveying fluid, for instance an effluent pump 26 controlling the flow rate in the effluent fluid line 13 within the fluid circuit 16. Also said pump is generally a peristaltic pump.

The disposable set 100 may further comprise one or more pressure detecting sites 48a, 49a, 50a, 52a, 60a (see figures 3 to 5) arranged on the blood circuit 17 and/or on the infusion line 63 and/or on the pre-blood-pump infusion line 51 and/or on the effluent line 13; the pressure detecting sites allow detecting a pressure in the respective line/circuit portion. In general, the pressure detecting sites 48a, 49a, 50a, 52a, 60a may be in the form of PODs (pressure oscillating diaphragms) that, once connected to the respective sensors on the extracorporeal blood treatment apparatus, allow to measure the pressure in the tube portion where the POD is placed. PODs placed on the blood circuit (or on tube section where blood may flow) are designed to eliminate the blood air interface, thereby reducing the risk of blood clotting.

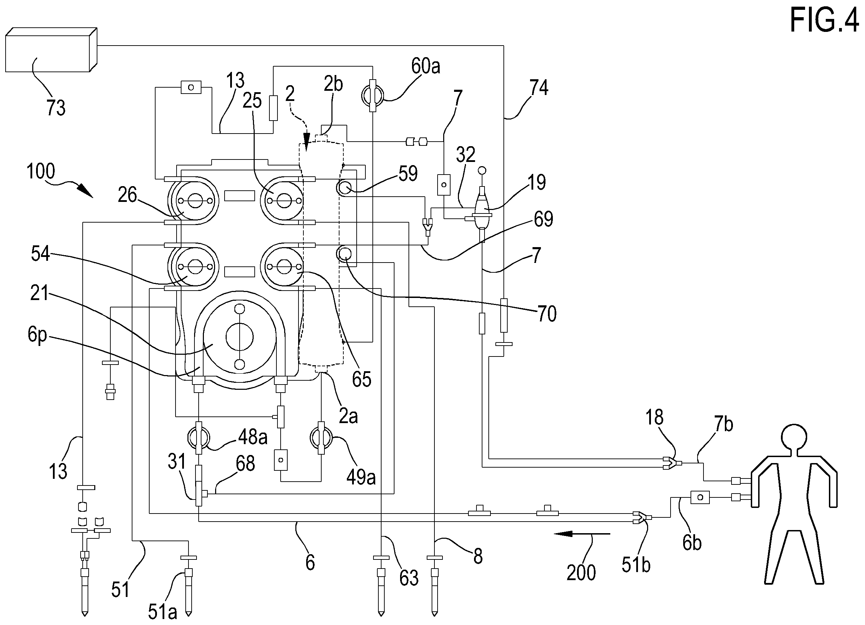

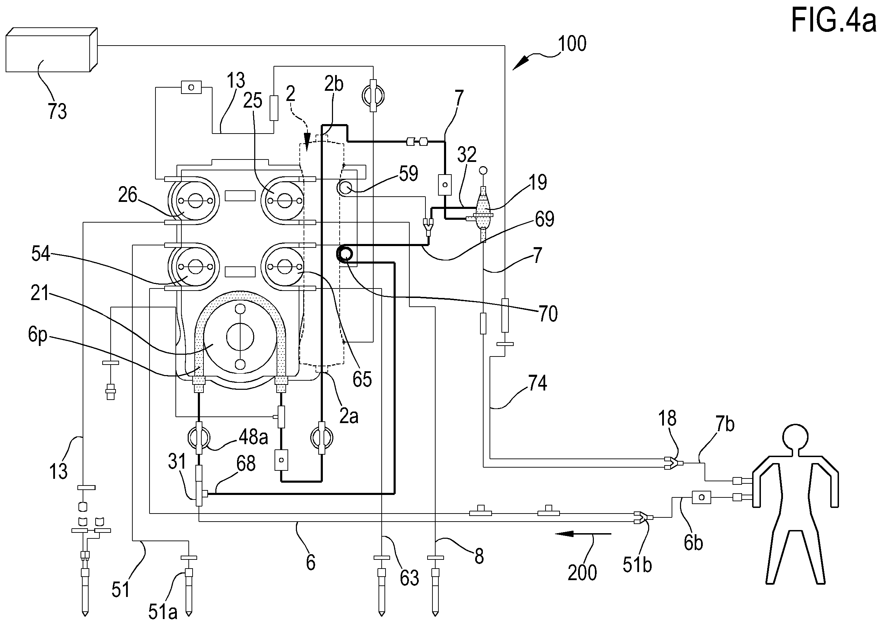

For example, a pressure detecting site 48a (e.g., a POD) may be arranged on the blood withdrawal line 6 between the first site 31 and the blood pump tract 6p, as shown in figures 3, 3a, 4, and 4a. An (additional) pressure detecting site 49a (e.g., a POD) may be arranged on the blood withdrawal line 6 between the blood pump tract 6p and the blood inlet 2a of the filtration unit 2. This is shown in all the figures 3 to 5.

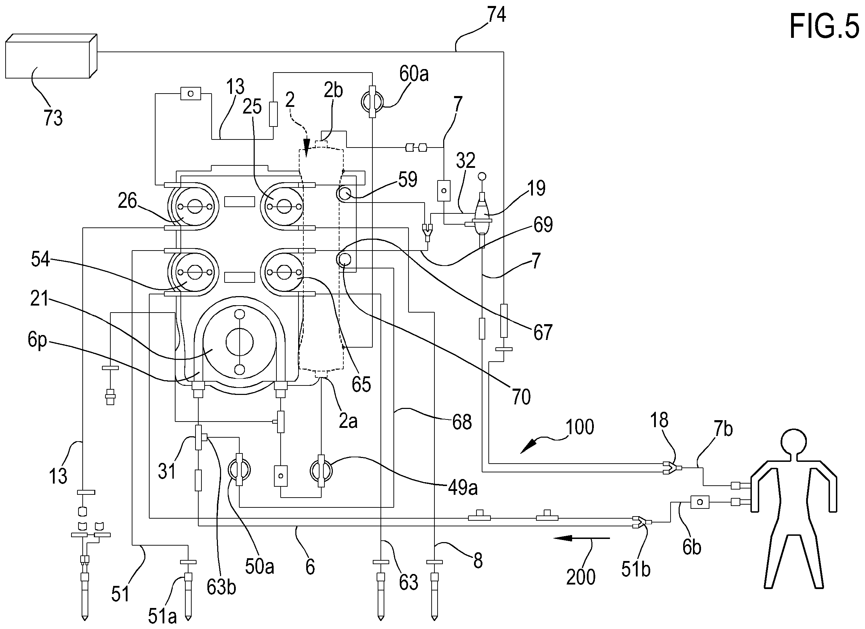

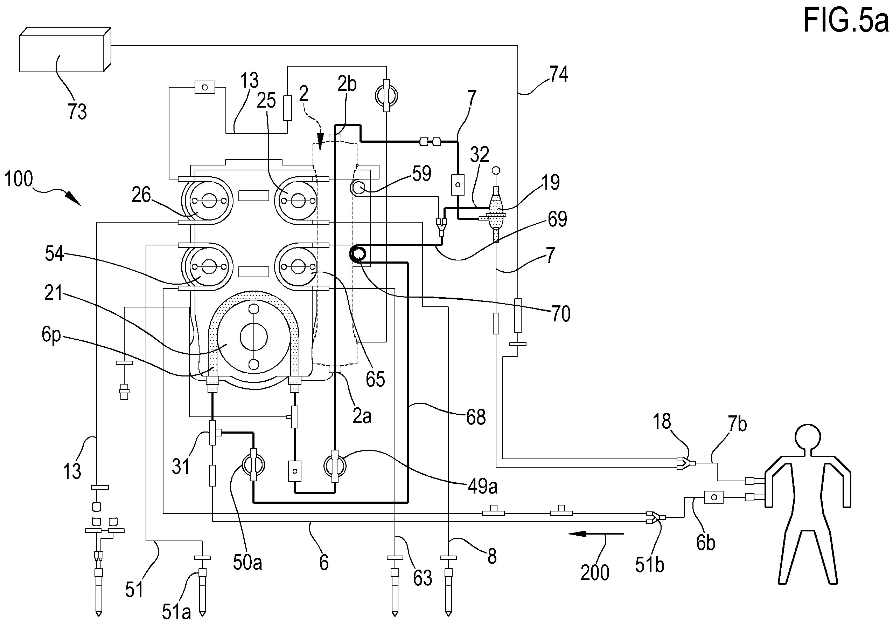

Alternatively to pressure detecting site 48a (or, in a non-shown embodiment, in addition to), a pressure detecting site 50a (e.g., a POD) may be arranged on the infusion line 63 close to, or at, the first site 31 (see figures 5 and 5a).

Alternatively to pressure detecting site 48a on the blood withdrawal line 6 or pressure detecting site 50a on the infusion line 63 (or, in a non-shown embodiment, in addition to one or both), a pressure detecting site 52a (e.g., a POD) may be arranged on the PBP infusion line 51 (see figures 3 and 3a).

The pressure detecting site of the disposable set may further comprise itself a pressure gauge, or alternatively may be configured to be connected to a pressure gauge configured to measure the blood or fluid pressure in the respective line.

Indeed, when the respective pressure site of the disposable set is properly connected to the pressure gauge of extracorporeal blood treatment apparatus, it defines a respective pressure sensor. In this regard, according to figures 1 and 1a, a first pressure sensor 48 is defined just upstream the blood pump 21 and a second pressure sensor 49 is defined downstream the blood pump 21 and upstream the filtration unit 2. An effluent sensor 60 is located in the effluent line 13 downstream the filtration unit 2 and upstream the effluent pump 26.

In the embodiment of figures 2 and 2a, pressure sensor 48 is removed and an additional pressure sensor 52 is located on the PBP infusion line 51 upstream the junction point 51c and downstream the PBP pump 54.

As an alternative to placing the sensor on the PBP infusion line 51, a pressure sensor 50 could be directly placed on the infusion line 63 and in particular at and immediately upstream of the junction point 51c. This latter configuration is shown in dashed lines.

The control unit 12 is connected to all sensor, actuators, and pumps and controls the operations of the apparatus.

### PRE-POST RECIRCULATION SEQUENCE

According to the present invention, the blood circuit 17 may be configured in a loop recirculation circuit 11 wherein blood may be recirculated in case e.g., an alarm is issued or e.g., an event occurs. From the structural point of view, in a first embodiment (e.g., figure 1a), the loop recirculation circuit 11 includes a delivery portion of the blood withdrawal line 6 interposed between the first site 31 of the blood withdrawal line 6 and the blood inlet 2a of the filtration unit 2 (and including the blood pump tract 6p), the blood chamber of the filtration unit 2, a receiving portion of the blood return line 7 interposed between the blood outlet 2b of the filtration unit 2 and the second site 32 of the blood return line 7 and a recirculation portion 66 of the infusion line 63 interposed between the first site 31 and the second site 32. More in detail, the recirculation portion 66 of the infusion line 63 comprises the second tract 68 and the third tract 69 of the infusion line 63 that are connected to each other at the branch site 67. The loop recirculation circuit 11 may further comprise the bubble trap 19 in case the third tract 69 enters into the bubble trap 19 for infusing into the blood return line 7. See figures 1a, 4a, and 5a.

In a second embodiment (e.g., see figure 2a), the loop recirculation circuit 11 includes a delivery portion of the blood withdrawal line 6 interposed between the first site 31 of the blood withdrawal line 6 and the blood inlet 2a of the filtration unit 2 (and including the blood pump tract 6p), the blood chamber of the filtration unit 2, a receiving portion of the blood return line 7 interposed between the blood outlet 2b of the filtration unit 2 and the second site 32 of the blood return line 7 and a recirculation portion 66 of the infusion line 63 interposed between the first site 31 and the PBP infusion line 51. More in detail, the recirculation portion 66 of the infusion line 63 comprises the second tract 68 and the third tract 69 of the infusion line 63 that are connected to each other at the branch site 67. Moreover, the loop recirculation circuit 11 comprises the common tract of the PBP infusion line 51 between the access point 51c and the second end 51b of the PBP infusion line 51. The loop recirculation circuit 11 may further comprise the bubble trap 19 in case the third tract 69 enters into the bubble trap 19 for infusing into the blood return line 7. See figures 2a and 3a.

During a recirculation condition, the loop recirculation circuit 11 is configured to allow blood to continuously flow within the loop recirculation circuit 11 in loop: the blood pump 21, engaged to the blood pump tract 6p, is configured to determine the blood flow within the loop recirculation circuit 11. The loop recirculation circuit is shown, with bold lines, in figures 1a, 2a, and according to specific embodiments of the disposable set 100, in figures 3a, 4a, and 5a.

The loop recirculation circuit 11 is configured to allow fluid recirculation, in a clockwise and/or in a counterclockwise direction, in order to avoid blood clogging in a situation wherein for example the blood pump has been temporarily stopped and the patient isolated as a reaction to an alarm or an event.

In general, blood recirculation in the loop recirculation circuit 11 may be started without stopping the blood pump 21. Indeed, it is sufficient that the control unit 12 sets the disposable in a configuration that allows the blood pushed by the blood pump 21 to recirculate in the loop recirculation circuit 11. It might be sufficient to allow blood to flow along the third tract 69 and the second tract 68 of the infusion line 63. To achieve this configuration, the selector 70 is moved to the recirculation position (neutral position). No infusion of fluid through infusion line 63 is commanded (i.e., infusion pump 65 is stopped).

Notably, the blood pump speed may be different during the recirculation procedure with respect to the blood pump speed during extracorporeal blood treatment.

As apparent, to achieve the blood recirculation in the loop recirculation circuit 11 it is not necessary to close any of the safety valves 20 and 27. Indeed, the safety valve/s 20, 27 on the blood circuit 17 may also be left open in case of total or partial access obstruction scenario; the control unit 12 still controls the blood pump 21 to determine blood flow within the loop recirculation circuit 11 of the blood circuit 17 of the disposable set, but additionally (in case of partial occlusion) a blood circulation is maintained in the blood circuit section between second site 32 and the patient and between the patient and the first site 31. Indeed, in the latter scenario, where the obstruction is not 'full/total', a small blood flow 'q' could be taken from the catheter at the second end 6b and the same small blood flow is returned to second end 7b, while a recirculation flow 'Q_{b}-q' (difference from the blood flow Qb defined by the blood pump action and the residual small flow q to and from the patient access) is present in the infusion line branches, namely in the second and third tracts 68 and 69.

Clearly, the alarm condition may trigger the need of blood pump stop and/or safety valve/s closure. Differently, blood pump stop may not be a must in a scenario where the recirculation is selected by the operator (versus alarm driven). In this case, it may be considered opening the intercepting element 70 before - optionally - close safety valves 20 and/or 27, and without stopping the blood pump 21.

Thus, the provided configuration allows keeping blood in movement, while the patient, if connected to the disposable set, may, or may not be isolated.

In order to allow recirculation of the fluid/blood in the loop recirculation circuit, the disposable set should not comprise or implement any component that may prevent recirculation, such as one-way valves in the infusion line/s, clamps, or selectors preventing or configured to prevent the blood flow in the loop.

According to the embodiments of figures 1, 1a, 4, 4a, and 5, and 5a the infusion line 63 is directly connected to the blood withdrawal line 6 at the first site 31. The term "*directly*" specifies that the infusion line 63 has its second end 63b fixed to the blood withdrawal line 6. Further, further branches are absent on the infusion line 63 between the infusion site 31 and the branch site 67.

According to the embodiments of figures 2, 2a, 3 and 3a, the infusion line 63 is not directly connected to the withdrawal line 6: the infusion line 63 shares indeed a common end portion with the pre-blood-pump infusion line 51. Thus said common end portion is configured to selectively transport the fluid solution coming from either the second fluid source 10, the fluid solution coming from the infusion fluid source 64, or blood during the blood recirculation condition.

During the recirculation condition, safety clamp/s 20, 27 on the blood withdrawal line 6 and/or on the blood return line 7 are optionally closed.

A default sequence may be initiated from an alarm state where blood pump is stopped and safety valve 20 or clamp on the blood return line 7 closed. In general, the infusion fluid source 64 (solution bag) should be available for the infusion line 63 e.g., on the replacement scale C. The sequence is very similar for all the shown embodiments.

The main steps of the sequence are the following: the control unit 12 moves the selector 70 to the recirculation position (neutral position); before moving the selector 70, the control unit 12 checks the bubble trap 19 fluid level and checks the access and return pressures to verify that they are compatible with moving the selector 70 to neutral position. If necessary, blood or fluid level in the bubble trap 19 is adjusted using the air pump 47.

Before re-starting the blood pump at a default low flow rate (usually different from the blood pump set during the extracorporeal blood treatment - blood flow rate is set from configuration menus), the control unit 12 checks the blood level in the bubble trap 19 to raise it in order to avoid recirculating air bubbles.

Note that in case vascular access to the patient (e.g., catheter access way) is obstructed, there might be a large pressure difference across the blood pump when the flow is stopped. Similar situation may occur during high flow rate therapies (such as ECCO2R) in the case where safety valves 20 and/or 27 are closed.

In case of large pressure differences across the blood pump, when selector 70 is suddenly moved to neutral, air bubbles may be drawn into the third tract 69 of the infusion line 63 (even if fluid level in the bubble trap 19 was OK before the selector 70 switch). To avoid the problem, the pressure difference (access vs return pressure) should be - at least partly - equalized before the selector 70 is moved.

Clearly, opening of safety valves 20 and 27 is an easy way, if considered safe given the occurring situation. However, in case of full vascular access obstruction, it might not work. As an alternative, the control unit 12 may infuse some infusion fluid e.g., from infusion fluid source 64 via infusion line 63 and/or from dialysis liquid source 14 via supply line 8 and post infusion line 58 or also across the filtration unit 2 towards the blood circuit 17 to adjust at least one of the access and return pressures as to bring them to a closer value range (e.g. within less than 50 or 100 mmHg of pressure difference). In this situation, the pressure equalization is a prerequisite to the move of the selector 70, rather than to the restart of the blood pump.

During the pre-post recirculation, the control unit 12 runs the blood pump usually, but not necessarily, in the same direction as during treatment. In particular, the control unit 12 keeps the blood pump running, while the operator intervenes and performs the troubleshooting.

Once operator intends to resume treatment, the control unit 12 asks for confirmation to resume the treatment or to change/discard the disposable set.

Once the operator confirms to resume treatment, the control unit 12 checks the blood level in the bubble trap 19 and provides an adjustment (e.g., via air pump 47) according to pressure level during recirculation versus last return pressure operating point prior to the recirculation.

Subsequently, the control unit 12 switches the selector 70 to pre-infusion position (first operating position) before restarting all pumps to their respective prescription settings. The control unit 12 controls the blood pump 21 to reach the prescribed blood flow rate.

Further, the control unit 12 is configured to perform a rinsing step of infusion line 63, and in particular of the second tract 68 of the infusion line 63 where blood was recirculated. In case the pre-infusion flow rate is too low for timely rinsing (or absent in the treatment settings, i.e., no pre-infusion), the control unit 12 commands a specific (higher) flow rate through the second tract 68 of the infusion line 63. Further, also the third tract 69 of the infusion line 63 is rinsed. The control unit 12 is configured to move the selector 70 to post-infusion (second operating position) and to wash blood away from the third tract 69.

Finally, in case the circuit according to figure 2 and 2a is used for recirculation, then also PBP line should be rinsed. In case PBP flow rate is not sufficient for rinsing, then the control unit 12 is configured to set a flow rate in the PBP line sufficient for the complete rinsing. Eventually, PBP flow rate and pre-infusion flow rate for rinsing are synchronized so that the common line tract is fully rinsed (e.g., without infusing too much citrate solution). Of course, if therapy prescription provides for post-infusion rather than pre-infusion, the control unit 12 switches the selector 70 to the second condition (post-infusion) after completion of the previously described rinsing steps.

## Claims

1. Disposable set (100) for an extracorporeal blood treatment apparatus (1) comprising:
- a filtration unit (2) having a blood inlet (2a) and a blood outlet (2b);
- a blood circuit (17) including:
➢ a blood withdrawal line (6) extending between a first end (6a) connected to the blood inlet (2a) of the filtration unit (2), and a second end (6b) for direct or indirect connection to a patient (P);
➢ a blood return line (7) extending between a first end (7a) connected to the blood outlet (2b) of the filtration unit (2), and a second end (7b) for direct or indirect connection to the patient;
➢ a blood pump tract (6p) located on the blood circuit (17) and configured to be engaged to a blood pump (21) of the extracorporeal blood treatment apparatus to promote blood flow in the blood circuit,
- an infusion line (63) extending from a first end (63a) for connection to an infusion fluid source (64),
- a pre-blood-pump infusion line (51) extending from a first end (51a) for connection to a second fluid source (10), and a second end (51b) fluidly connected to the blood withdrawal line (6) between the second end (6b) of the blood withdrawal line (6) and the blood pump tract (6p),
wherein the infusion line (63) branches off at a branch site (67) towards:
➢ a second end (63b) of the infusion line (63); and
➢ a third end (63c) of the infusion line (63) in direct or indirect fluid communication with the blood return line (7) at a second site (32), the second site (32) being interposed between the blood pump tract (6p) and the second end of the blood return line (7);
**characterized in that** the second end (63b) of the infusion line (63) is directly connected to the pre-blood-pump infusion line (51) and in fluid communication **with** the blood withdrawal line (6) at the first site (31), the first site (31) being interposed between the second end (6b) of the blood withdrawal line (6) and the blood pump tract (6p);
wherein the infusion line (63) and the pre-blood-pump infusion line (51) are connected together and present a common infusion tract extending up to the first site (31) of the blood withdrawal line (6), wherein the blood circuit is designed to allow fluid flow in a loop recirculation circuit (11) including:
- a portion of the blood withdrawal line (6) interposed between the first site (31) of the blood withdrawal line (6) and the blood inlet (2a) of the filtration unit (2);
- the blood pump tract (6p);
- the filtration unit (2);
- a portion of the blood return line (7) interposed between the blood outlet (2b) of the filtration unit (2) and the second site (32) of the blood return line (7);
- a recirculation portion (66) of the infusion line (63) downstream the branch site (67) and interposed between the first site (31) and the second site (32);
- the common infusion tract;
the loop recirculation circuit (11) being configured to allow blood or a fluid, at least in a recirculation condition, to continuously flow in a loop circulation.

2. Disposable set according to the preceding claim 1, wherein the loop recirculation circuit (11) is configured to allow fluid recirculation in a first direction from the first site (31) towards the filtration unit (2) and from the filtration unit (2) towards the second site (32) or in a second direction from the second site (32) towards the filtration unit (2) and from the filtration unit (2) towards the first site (31), and wherein:
- the blood inlet (2a) of the filtration unit (2) is in bidirectional fluid communication with the recirculation portion (66) of the infusion line (63) through the first site (31); and
- the blood outlet (2b) of the filtration unit (2) is in bidirectional fluid communication with the recirculation portion (66) of the infusion line (63) through the second site (32).

3. Disposable set according to any one of the preceding claims 1 and 2, wherein
the first site (31) comprises a fluid connector, in particular a three-way fluid connector, including:
- a blood inlet fluidly connected to the withdrawal line (6) and facing the second end (6b) of the blood withdrawal line (6);
- a blood outlet fluidly connected to the withdrawal line (6) and facing the first end (6a) of the blood withdrawal line (6), optionally facing the pump tract (6p) of the blood withdrawal line (6);
- an infusion inlet fluidly connected to the recirculation portion (66) of the infusion line (63).

4. Disposable set according to any one of the preceding claims 1 to 3, further comprising a blood warmer disposable unit comprising a blood flow path with an inlet and an outlet, the blood warmer disposable unit being fluidly connected the blood circuit (17) upstream the second site (32), in particular, the inlet of the blood warmer disposable unit being fluidly connected to the blood return line (7) and the outlet of the blood warmer disposable unit being fluidly connected to the blood return line (7) upstream the second site (32).

5. Disposable set according to any one of the preceding claims 1 to 4, wherein the infusion line (63) extends from the first end (63a) of the infusion line (63) for a first tract (72), said first tract (72) branching at the branch site (67) in:
- a second tract (68) extending from the branch site (67) up to the second end (63b) of the infusion line (63); and
- a third tract (69) extending from the branch site (67) up to the third end (63c) of the infusion line (63),
the recirculation portion (66) of the infusion line (63) comprising the second tract (68) and the third tract (69) of the infusion line (63),
wherein the infusion line (63) comprises a respective infusion pump tract (65p) arranged on the first tract (72) of the infusion line (63) and configured to be engaged by an infusion pump (65) of the extracorporeal blood treatment apparatus, said infusion pump tract (65p) being interposed between the first end (63a) and the branch site (67) of the infusion line (63).

6. Disposable set according to any one of the preceding claims 1 to 5, wherein the third end (63c) of the infusion line (63) is directly connected to the blood return line (7) at the second site (32) or
wherein the disposable set comprises a bubble trap (19) configured to remove bubbles from the blood during the extracorporeal blood treatment and arranged on the return line (7) of the blood circuit (17), the second site (32) being arranged at the bubble trap (19), and the third end (63c) of the infusion line (63) being fluidly connected to the bubble trap (19), and wherein the loop recirculation circuit (11) further comprises said bubble trap (19).

7. Disposable set according to the preceding claim 6, wherein the bubble trap (19) comprises:
- a blood inlet connected to the receiving portion of the blood return line (7), the receiving portion of the return line (7) being interposed between the outlet (2b) of the filtration unit (2) and said blood inlet of the bubble trap (19);
- a blood outlet connected to a secondary tract of the blood return line (7), the secondary tract of the blood return line (7) being interposed between said blood outlet of the bubble trap (19) and the second end (7b) of the return line (7);
- an auxiliary connection connected to the third end (63c) of the infusion line (63).

8. Disposable set according to any one of the preceding claims 1 to 7, wherein the pre-blood-pump (PBP) infusion
line (51) comprises a respective PBP infusion pump tract (51p) configured to be engaged by a PBP infusion pump (54),
wherein the second end (63b) of the infusion line (63) is directly connected to the pre-blood-pump infusion line (51) downstream the PBP infusion pump tract (51p).

9. Disposable set according to any one of the preceding claims 1 to 8, further comprising a pressure detecting site (52a) arranged on the pre-blood-pump infusion line (51) between a PBP infusion pump tract (51p) of the pre-blood-pump infusion line (51) and the second end (51b) of the pre-blood-pump infusion line (51), wherein the pressure detecting site (52a) is arranged between the PBP infusion pump tract (51p) and the second end (63b) of the infusion line (63), in particular said pressure detecting site (52a) being a pressure pod.

10. Disposable set according to any one of the preceding claims 1 to 9, wherein the filtration unit (2) comprises:
➢ a primary chamber (3) fluidly connected to the blood circuit through the blood inlet and the blood outlet of the filtration unit (2),
➢ a secondary chamber (4) fluidly connected or connectable to a fluid circuit (16), said secondary chamber (4) being configured to receive dialysis fluid and/or to remove effluent fluid,
and wherein the primary chamber (3) is separated from the secondary chamber (4) by a semi-permeable membrane (5),
the disposable set further comprising a fluid circuit (16) including:
- an effluent fluid line (13) extending between a first end for connection to a discharge unit, such as a collection container (62), and a second end fluidly connected to a fluid outlet of a secondary chamber (4) of the filtration unit (2), wherein said effluent fluid line (13) comprises an effluent pump tract (13p) configured to be engaged to an effluent pump (26) of the extracorporeal blood treatment apparatus to promote flow of effluent fluid from the fluid outlet of the secondary chamber (4) towards the discharge unit;
- optionally a fluid supply line (8) extending between a first end for connection to a dialysis fluid source (14), and a second end fluidly connected to a fluid inlet of a secondary chamber (4) of the filtration unit (2), wherein said fluid supply line (8) comprises a supply pump tract (8p) configured to be engaged to a supply pump (25) of the extracorporeal blood treatment apparatus to promote dialysis fluid towards the fluid inlet of the secondary chamber (4) of the filtration unit (2).

11. Extracorporeal blood treatment apparatus including:
- one or more pumps (21, 25, 26, 54, 65, 75) comprising a blood pump (21),
- one or more intercepting elements (20, 27, 59, 70),
- a disposable set (100) in accordance with any one of the previous claims
- a control unit (12) configured to control said one or more pumps (21, 25, 26, 54, 65, 75) and said one or more intercepting elements (20, 27, 59, 70) in order to define different flow circuits inside the blood circuit (17) and the infusion line (63), the control unit (12) being configured to define at least the following flow circuits:
➢ a treatment flow circuit, wherein the one or more pumps (21, 25, 26, 54, 65, 75) and the one or more intercepting elements (20, 27, 59, 70) are configured to allow blood to flow from the second end (6b) of the blood withdrawal line (6) to the filtration unit (2) and from the filtration unit (2) to the second end (7b) of the blood return line (7); and
➢ a loop recirculation circuit (11), wherein the one or more pumps (21, 25, 26, 54, 65, 75) and the one or more intercepting elements (20, 27, 59, 70) are configured to allow blood or a fluid to continuously flow in a loop circulation,
wherein the infusion line (63) and the pre-blood-pump infusion line (51) are connected together and present a common infusion tract extending up to the first site (31) of the blood withdrawal line (6), wherein the loop recirculation circuit (11) includes:
• a portion of the blood withdrawal line (6) interposed between the first site (31) of the blood withdrawal line (6) and the blood inlet (2a) of the filtration unit (2);
• the blood pump tract (6p);
• the filtration unit (2);
• a portion of the blood return line (7) interposed between the blood outlet (2b) of the filtration unit (2) and the second site (32) of the blood return line (7);
• a recirculation portion (66) of the infusion line (63) downstream the branch site (67) and interposed between the first site (31) and the second site (32);
• the common infusion tract.

12. Apparatus according to the preceding claim 11, wherein the control unit (12) is configured to perform a recirculation procedure comprising the steps of:
- defining the loop recirculation circuit (11) by at least:
➢ commanding the one or more intercepting elements (20, 27, 59, 70) to put in fluid communication, the first site (31) with the second site (32) through the recirculation portion (66) of the infusion line (63);
- controlling the blood pump (21) to determine blood flow within the loop recirculation circuit (11) of the blood circuit (17) of the disposable set,
in particular wherein the one or more intercepting elements (20, 27, 59, 70) comprise an intercepting element (70) acting on the infusion line (63) and movable between:
➢ a recirculation position, wherein the intercepting element (70) is configured to allow fluid communication between the first site (31) and the second site (32) through the recirculation portion (63) of the infusion line (63);
➢ a lock position, wherein the intercepting element (70) is configured to prevent fluid communication between the first site (31) and the second site (32) through the recirculation portion (63) of the infusion line (63);
and wherein, in the recirculation procedure, the control unit (12) is configured to switch the intercepting element (70) to the recirculation position.

13. Apparatus according to claim 12, wherein, before starting the recirculation procedure, the control unit (12) is configured to:
a. stop the blood pump (21);
b. prior switching the intercepting element (70) to the recirculation position, operate on the blood circuit (17) to reduce a pressure difference between portions of the blood circuit (17) across the blood pump (21);
in particular wherein to reduce the pressure difference, the control unit (12) is configured to perform at least one of the following actions:
i. commanding the one or more intercepting elements (20, 27, 59, 70) from a close to an open position to switch from preventing to allowing fluid passage in the blood withdrawal line (6) between the second end (6b) of the blood withdrawal line (6) and the first site (31) and/or in the blood return line (7) between the second site (32) and the second end (7b) of the blood return line (7);
ii. infusing an infusion fluid into the blood circuit (17) to adjust at least one of an access pressure and a return pressure as to bring them to a closer value, for example to a pressure difference of less than 100 mmHg.

14. Apparatus according to any one of the preceding claims 11 to 13, wherein one or more pumps (21, 25, 26, 54, 65, 75) comprises an infusion pump (65) active on the infusion pump tract (63p) of the infusion line, wherein, in the recirculation procedure, the control unit (12) is configured to:
a. keep said infusion pump (65) inactive, in particular the infusion pump (65) being an occlusive pump, such as a peristaltic pump;
b. stop any ultrafiltration of fluids from a primary chamber (3) towards a secondary chamber (4) of the filtration unit (2);
c. recirculate blood in the loop recirculation circuit (11) to prevent blood from clotting;
d. optionally run the blood pump (21) at a speed different from a speed of the blood pump (21) during the extracorporeal blood treatment, in particular, during the recirculation procedure, the control unit (12) is configured to run the blood pump (21) in a same direction as during the extracorporeal blood treatment.

15. Apparatus according to any one of the preceding claims 11 to 14, wherein, at an end of the recirculation procedure, the control unit (12) is configured to:
a. move an intercepting element (70) acting on the infusion line (63) to a lock position allowing fluid flow in a second tract (68) extending from the branch site (67) up to the second end (63b) of the infusion line (63) and blocking a fluid flow in a third tract (69) extending from the branch site (67) up to the third end (63c) of the infusion line (63) to prevent fluid communication between the first site (31) and the second site (32) through the recirculation portion (66);
b. rinse the blood in the second tract (68) with a fluid from the infusion line (63).

## Patentansprüche

1. Einwegset (100) für eine extrakorporale Blutbehandlungsvorrichtung (1), umfassend:
- eine Filtrationseinheit (2) mit einem Bluteinlass (2a) und einem Blutauslass (2b);
- einen Blutkreislauf (17), welcher einschließt:
➢ eine Blutentnahmeleitung (6), die sich zwischen einem ersten Ende (6a), das mit dem Bluteinlass (2a) der Filtrationseinheit (2) verbunden ist, und einem zweiten Ende (6b) zur direkten oder indirekten Verbindung mit einem Patienten (P) befindet;
➢ eine Blutrückführleitung (7), die sich zwischen einem ersten Ende (7a), das mit dem Blutauslass (2b) der Filtrationseinheit (2) verbunden ist, und einem zweiten Ende (7b) zur direkten oder indirekten Verbindung mit dem Patienten befindet;
➢ einen Blutpumpentrakt (6p), der sich an dem Blutkreislauf (17) befindet und ausgestaltet ist, um in Eingriff mit einer Blutpumpe (21) der extrakorporalen Blutbehandlungsvorrichtung zu kommen, um Blutfluss in dem Blutkreislauf zu fördern,
- eine Infusionsleitung (63), die sich von einem ersten Ende (63a) zur Verbindung mit einer Infusionsfluidquelle (64) erstreckt,
- eine Vorblutpumpen-Infusionsleitung (51), die sich von einem ersten Ende (51a) zur Verbindung mit einer zweiten Fluidquelle (10) und einem zweiten Ende (51b) erstreckt, das fluidtechnisch mit der Blutentnahmeleitung (6) zwischen dem zweiten Ende (6b) der Blutentnahmeleitung (6) und dem Blutpumpentrakt (6p) verbunden ist,
wobei die Infusionsleitung (63) an einer Verzweigungsstelle (67) verzweigt zu:
➢ einem zweiten Ende (63b) der Infusionsleitung (63);
➢ einem dritten Ende (63c) der Infusionsleitung (63) in direkter oder indirekter Fluidkommunikation mit der Blutrückführleitung (7) an einer zweiten Stelle (32),
wobei die zweite Stelle (32) zwischen dem Blutpumpentrakt (6p) und dem zweiten Ende der Blutrückführleitung (7) angeordnet ist;
**dadurch gekennzeichnet, dass** das zweite Ende (63b) der Infusionsleitung (63) direkt mit der Vorblutpumpen-Infusionsleitung (51) und in Fluidkommunikation mit der Blutentnahmeleitung (6) an der ersten Stelle (31) verbunden ist, wobei die erste Stelle (31) zwischen dem zweiten Ende (6b) der Blutentnahmeleitung (6) und dem Blutpumpentrakt (6p) angeordnet ist;
wobei die Infusionsleitung (63) und die Vorblutpumpen-Infusionsleitung (51) miteinander verbunden sind und einen gemeinsamen Infusionstrakt darstellen, der sich bis zu der ersten Stelle (31) der Blutentnahmeleitung (6) erstreckt, wobei der Blutkreislauf konzipiert ist, um Fluidfluss in einem Schleifenrezirkulationskreislauf (11) zuzulassen, welcher einschließt:
- einen Teil der Blutentnahmeleitung (6), der zwischen der ersten Stelle (31) der Blutentnahmeleitung (6) und dem Bluteinlass (2a) der Filtrationseinheit (2) angeordnet ist;
- den Blutpumpentrakt (6p);
- die Filtrationseinheit (2);
- einen Teil der Blutrückführleitung (7), der zwischen dem Blutauslass (2b) der Filtrationseinheit (2) und der zweiten Stelle (32) der Blutrückführleitung (7) angeordnet ist;
- einen Rezirkulationsteil (66) der Infusionsleitung (63), welcher stromabwärts der Verzweigungsstelle (67) und zwischen der ersten Stelle (31) und der zweiten Stelle (32) angeordnet ist;
- den gemeinsamen Infusionstrakt;
wobei der Schleifenrezirkulationskreislauf (11) so ausgestaltet ist, dass Blut oder ein Fluid mindestens in einem Rezirkulationszustand kontinuierlich in einem Schleifenkreislauf fließen kann.

2. Einwegset nach dem vorhergehenden Anspruch 1, wobei der Schleifenrezirkulationskreislauf (11) ausgestaltet ist, um Fluidrezirkulation in einer ersten Richtung von der ersten Stelle (31) zu der Filtrationseinheit (2) und von der Filtrationseinheit (2) zu der zweiten Stelle (32) oder in einer zweiten Richtung von der zweiten Stelle (32) zu der Filtrationseinheit (2) und von der Filtrationseinheit (2) zu der ersten Stelle (31) zuzulassen, und wobei:
- der Bluteinlass (2a) der Filtrationseinheit (2) durch die erste Stelle (31) hindurch in bidirektionaler Fluidkommunikation mit dem Rezirkulationsteil (66) der Infusionsleitung (63) ist; und
- der Blutauslass (2b) der Filtrationseinheit (2) durch die zweite Stelle (32) hindurch in bidirektionaler Fluidkommunikation mit dem Rezirkulationsteil (66) der Infusionsleitung (63) ist.

3. Einwegset nach einem der vorhergehenden Ansprüche 1 und 2, wobei
die erste Stelle (31) einen Fluidverbinder umfasst, insbesondere einen Dreiwege-Fluidverbinder, welcher einschließt:
- einen Bluteinlass, der fluidtechnisch mit der Entnahmeleitung (6) verbunden ist und zu dem zweiten Ende (6b) der Blutentnahmeleitung (6) weist;
- einen Blutauslass, der fluidtechnisch mit der Entnahmeleitung (6) verbunden ist und zu dem ersten Ende (6a) der Blutentnahmeleitung (6) weist, gegebenenfalls zu dem Pumpentrakt (6p) der Blutentnahmeleitung (6) weist;
- einen Infusionseinlass, der fluidtechnisch mit dem Rezirkulationsteil (66) der Infusionsleitung (63) verbunden ist.

4. Einwegset nach einem der vorhergehenden Ansprüche 1 bis 3, des Weiteren umfassend eine Blutwärmer-Einwegeinheit, umfassend einen Blutflusspfad mit einem Einlass und einem Auslass, wobei die Blutwärmer-Einwegeinheit fluidtechnisch mit dem Blutkreislauf (17) stromaufwärts der zweiten Stelle (32) verbunden ist, wobei der Einlass der Blutwärmer-Einwegeinheit insbesondere fluidtechnisch mit der Blutrückführleitung (7) verbunden ist, und der Auslass der Blutwärmer-Einwegeinheit fluidtechnisch mit der Blutrückführleitung (7) stromaufwärts der zweiten Stelle (32) verbunden ist.

5. Einwegset nach einem der vorhergehenden Ansprüche 1 bis 4, wobei die Infusionsleitung (63) sich von dem ersten Ende (63a) der Infusionsleitung (63) für einen ersten Trakt (72) erstreckt, wobei der erste Trakt (72) an der Verzweigungsstelle (67) verzweigt in:
- einen zweiten Trakt (68), der sich von der Verzweigungsstelle (67) bis zu dem zweiten Ende (63b) der Infusionsleitung (63) erstreckt; und
- einen dritten Trakt (69), der sich von der Verzweigungsstelle (67) bis zu dem dritten Ende (63c) der Infusionsleitung (63) erstreckt,
wobei der Rezirkulationsteil (66) der Infusionsleitung (63) den zweiten Trakt (68) und den dritten Trakt (69) der Infusionsleitung (63) umfasst,
wobei die Infusionsleitung (63) einen jeweiligen Infusionspumpentrakt (65p) umfasst, der auf dem ersten Trakt (72) der Infusionsleitung (63) angeordnet ist und ausgestaltet ist, um in Eingriff mit einer Infusionspumpe (65) der extrakorporalen Blutbehandlungsvorrichtung zu kommen, wobei der Infusionspumpentrakt (65p) zwischen dem ersten Ende (63a) und der Verzweigungsstelle (67) der Infusionsleitung (63) angeordnet ist.

6. Einwegset nach einem der vorhergehenden Ansprüche 1 bis 5, wobei das dritte Ende (63c) der Infusionsleitung (63) direkt mit der Blutrückführleitung (7) an der zweiten Stelle (32) verbunden ist, oder
wobei das Einwegset einen Blasenfänger (19) umfasst, der ausgestaltet ist, um während der extrakorporalen Blutbehandlung Blasen aus dem Blut zu entfernen, und an der Rückführleitung (7) des Blutkreislaufs (17) angeordnet ist, wobei die zweite Stelle (32) an dem Blasenfänger (19) angeordnet ist, und das dritte Ende (63c) der Infusionsleitung (63) fluidtechnisch mit dem Blasenfänger (19) verbunden ist, und wobei der Schleifenrezirkulationskreislauf (11) des Weiteren den Blasenfänger (19) umfasst.

7. Einwegset nach dem vorhergehenden Anspruch 6, wobei der Blasenfänger (19) umfasst:
- einen Bluteinlass, der mit dem Empfangsteil der Blutrückführleitung (7) verbunden ist, wobei der Empfangsteil der Rückführleitung (7) zwischen dem Auslass (2b) der Filtrationseinheit (2) und dem Bluteinlass des Blasenfängers (19) angeordnet ist;
- einen Blutauslass, der mit einem Sekundärtrakt der Blutrückführleitung (7) verbunden ist, wobei der Sekundärtrakt der Blutrückführleitung (7) zwischen dem Blutauslass des Blasenfängers (19) und dem zweiten Ende (7b) der Rückführleitung (7) angeordnet ist;
- eine Hilfsverbindung, die mit dem dritten Ende (63c) der Infusionsleitung (63) verbunden ist.

8. Einwegset nach einem der vorhergehenden Ansprüche 1 bis 7, wobei die Vorblutpumpen-(PBP)-Infusionsleitung (51) einen jeweiligen PBP-Infusionspumpentrakt (51p) umfasst, der ausgestaltet ist, um in Eingriff mit einer PBP-Infusionspumpe (54) zu kommen,
wobei das zweite Ende (63b) der Infusionsleitung (63) direkt mit der Vorblutpumpen-Infusionsleitung (51) stromabwärts des PBP-Infusionspumpentrakts (51p) verbunden ist.

9. Einwegset nach einem der vorhergehenden Ansprüche 1 bis 8, des Weiteren umfassend eine Druckdetektionsstelle (52a), die an der Vorblutpumpen-Infusionsleitung (51) zwischen einem PBP-Infusionspumpentrakt (51p) der Vorblutpumpen-Infusionsleitung (51) und dem zweiten Ende (51b) der Vorblutpumpen-Infusionsleitung (51) angeordnet ist, wobei die Druckdetektionsstelle (52a) zwischen dem PBP-Infusionspumpentrakt (51p) und dem zweiten Ende (63b) der Infusionsleitung (63) angeordnet ist, wobei insbesondere die Druckdetektionsstelle (52a) eine Druckkapsel (Druck-POD) ist.

10. Einwegset nach einem der vorhergehenden Ansprüche 1 bis 9, wobei die Filtrationseinheit (2) umfasst:
➢ eine Primärkammer (3), die fluidtechnisch durch den Bluteinlass und den Blutauslass der Filtrationseinheit (2) hindurch mit dem Blutkreislauf verbunden ist,
➢ eine Sekundärkammer (4), die fluidtechnisch mit einem Fluidkreislauf (16) verbunden oder verbindbar ist, wobei die Sekundärkammer (4) ausgestaltet ist, um Dialysefluid zu empfangen und/oder um Ausflussfluid zu entfernen,
und wobei die Primärkammer (3) von der Sekundärkammer (4) durch eine semipermeable Membran (5) getrennt ist,
wobei das Einwegset des Weiteren einen Fluidkreislauf (16) umfasst, der Folgendes einschließt:
- eine Abflussfluidleitung (13), die sich zwischen einem ersten Ende zur Verbindung mit einer Austragungseinheit, wie einem Sammelbehälter (62), und einem zweiten Ende erstreckt, das fluidtechnisch mit einem Fluidauslass einer Sekundärkammer (4) der Filtrationseinheit (2) verbunden ist, wobei die Abflussfluidleitung (13) einen Abflusspumpentrakt (13p) umfasst, der ausgestaltet ist, um in Eingriff mit einer Abflusspumpe (26) der extrakorporalen Blutbehandlungsvorrichtung zu kommen, um Fluss des Abflussfluids aus dem Fluidauslass der Sekundärkammer (4) zu der Austragungseinheit zu fördern;
- gegebenenfalls eine Fluidzufuhrleitung (8), die sich zwischen einem ersten Ende zur Verbindung mit einer Dialysefluidquelle (14) und einem zweiten Ende erstreckt, das fluidtechnisch mit einem Fluideinlass einer Sekundärkammer (4) der Filtrationseinheit (2) verbunden ist, wobei die Fluidzufuhrleitung (8) einen Zufuhrpumpentrakt (8p) umfasst, der ausgestaltet ist, um in Eingriff mit einer Zufuhrpumpe (25) der extrakorporalen Blutbehandlungsvorrichtung zu kommen, um Dialysefluid zu dem Fluideinlass der Sekundärkammer (4) der Filtrationseinheit (2) zu fördern.

11. Extrakorporale Blutbehandlungsvorrichtung, welche Folgendes einschließt:
- eine oder mehrere Pumpen (21, 25, 26, 54, 65, 75), umfassend eine Blutpumpe (21),
- ein oder mehrere Abstellelemente (20, 27, 59, 70),
- ein Einwegset (100) gemäß einem der vorhergehenden Ansprüche,
- eine Steuereinheit (12), die ausgestaltet ist, um die eine oder mehreren Pumpen (21, 25, 26, 54, 65, 75) und das eine oder die mehreren Abstellelemente (20, 27, 59, 70) zu steuern, um unterschiedliche Flusskreisläufe innerhalb des Blutkreislaufs (17) und der Infusionsleitung (63) zu definieren, wobei die Steuereinheit (12) ausgestaltet ist, um mindestens die folgenden Flusskreisläufe zu definieren:
➢ einen Behandlungsflusskreislauf, wobei die eine oder mehreren Pumpen (21, 25, 26, 54, 65, 75) und das eine oder die mehreren Abstellelemente (20, 27, 59, 70) ausgestaltet sind, um Blut von dem zweiten Ende (6b) der Blutentnahmeleitung (6) zu der Filtrationseinheit (2) und von der Filtrationseinheit (2) zu dem zweiten Ende (7b) der Blutrückführleitung (7) fließen zu lassen; und
➢ einen Schleifenrezirkulationskreislauf (11), wobei die eine oder mehreren Pumpen (21, 25, 26, 54, 65, 75) und das eine oder die mehreren Abstellelemente (20, 27, 59, 70) ausgestaltet sind, um Blut oder ein Fluid kontinuierlich in einem Schleifenkreislauf fließen zu lassen,
wobei die Infusionsleitung (63) und die Vorblutpumpen-Infusionsleitung (51) miteinander verbunden sind und einen gemeinsamen Infusionstrakt darstellen, der sich bis zu der ersten Stelle (31) der Blutentnahmeleitung (6) erstreckt, wobei der Schleifenrezirkulationskreislauf (11) einschließt:
• einen Teil der Blutentnahmeleitung (6), der zwischen der ersten Stelle (31) der Blutentnahmeleitung (6) und dem Bluteinlass (2a) der Filtrationseinheit (2) angeordnet ist;
• den Blutpumpentrakt (6p);
• die Filtrationseinheit (2);
• einen Teil der Blutrückführleitung (7), der zwischen dem Blutauslass (2b) der Filtrationseinheit (2) und der zweiten Stelle (32) der Blutrückführleitung (7) angeordnet ist;
• einen Rezirkulationsteil (66) der Infusionsleitung (63), welcher stromabwärts der Verzweigungsstelle (67) und zwischen der ersten Stelle (31) und der zweiten Stelle (32) angeordnet ist;
• den gemeinsamen Infusionstrakt.

12. Vorrichtung nach dem vorhergehenden Anspruch 11, wobei die Steuereinheit (12) ausgestaltet ist, um eine Rezirkulationsprozedur durchzuführen, welche die folgenden Schritte umfasst:
- Definieren des Schleifenrezirkulationskreislaufs (11) durch mindestens eines von:
➢ Befehlen dem einen oder den mehreren Abstellelementen (20, 27, 59, 70), die erste Stelle (31) mit der zweiten Stelle (32) durch den Rezirkulationsteil (66) der Infusionsleitung (63) in Fluidkommunikation zu versetzen;
- Steuern der Blutpumpe (21), um Blutfluss innerhalb des Schleifenrezirkulationskreislaufs (11) des Blutkreislaufs (17) des Einwegsets zu bestimmen,
wobei insbesondere das eine oder die mehreren Abstellelemente (20, 27, 59, 70) ein Abstellelement (70) umfassen, das auf die Infusionsleitung (63) wirkt und beweglich ist zwischen:
➢ einer Rezirkulationsposition, wobei das Abstellelement (70) ausgestaltet ist, um Fluidkommunikation zwischen der ersten Stelle (31) und der zweiten Stelle (32) durch den Rezirkulationsteil (63) der Infusionsleitung (63) zuzulassen;
➢ einer Sperrposition, wobei das Abstellelement (70) ausgestaltet ist, um Fluidkommunikation zwischen der ersten Stelle (31) und der zweiten Stelle (32) durch den Rezirkulationsteil (63) der Infusionsleitung (63) zu verhindern;
und wobei die Steuereinheit (12) ausgestaltet ist, um in der Rezirkulationsprozedur das Abstellelement (70) in die Rezirkulationsposition zu schalten.

13. Vorrichtung nach Anspruch 12, wobei die Steuereinheit (12), bevor die Rezirkulationsprozedur startet, ausgestaltet ist zum:
a. Stoppen der Blutpumpe (21);
b. Einwirken auf den Blutkreislauf (17) vor dem Schalten des Abstellelements (70) in die Rezirkulationsposition, um eine Druckdifferenz zwischen Teilen des Blutkreislaufs (17) über der Blutpumpe (21) zu reduzieren;
wobei die Steuereinheit (12) insbesondere zum Reduzieren der Druckdifferenz ausgestaltet ist, um mindestens eine der folgenden Aktionen durchzuführen:
i. Befehlen des einen oder der mehreren Abstellelemente (20, 27, 59, 70) aus einer geschlossenen in eine offene Position, um von dem Verhindern zum Zulassen von Fluiddurchgang in der Blutentnahmeleitung (6) zwischen dem zweiten Ende (6b) der Blutentnahmeleitung (6) und der ersten Stelle (31) und/oder in der Blutrückführleitung (7) zwischen der zweiten Stelle (32) und dem zweiten Ende (7b) der Blutrückführleitung (7) umzuschalten;
ii. Infundieren eines Infusionsfluids in den Blutkreislauf (17), um mindestens einen von einem Zugangsdruck und einem Rückführdruck anzupassen, um sie auf einen näher beieinander liegenden Wert zu bringen, beispielsweise auf eine Druckdifferenz von weniger als 100 mmHg.

14. Vorrichtung nach einem der vorhergehenden Ansprüche 11 bis 13, wobei eine oder mehrere Pumpen (21, 25, 26, 54, 65, 75) eine Infusionspumpe (65) umfasst, die an dem Infusionspumpentrakt (63p) der Infusionsleitung aktiv ist,
wobei die Steuereinheit (12) in der Rezirkulationsprozedur ausgestaltet ist zum:
a. Inaktivhalten der Infusionspumpe (65), wobei insbesondere die Infusionspumpe (65) eine okklusive Pumpe ist, wie eine Peristaltikpumpe;
b. Stoppen von jeglicher Ultrafiltration von Fluiden von einer Primärkammer (3) zu einer Sekundärkammer (4) der Filtrationseinheit (2);
c. Rezirkulieren von Blut in dem Schleifenrezirkulationskreislauf (11), um Blut am Gerinnen zu hindern;
d. gegebenenfalls Laufenlassen der Blutpumpe (21) mit einer Geschwindigkeit, die sich von einer Geschwindigkeit der Blutpumpe (21) während der extrakorporalen Blutbehandlung, insbesondere während der Rezirkulationsprozedur, unterscheidet, wobei die Steuereinheit (12) ausgestaltet ist, um die Blutpumpe (21) in derselben Richtung wie während der extrakorporalen Blutbehandlung laufen zu lassen.

15. Vorrichtung nach einem der vorhergehenden Ansprüche 11 bis 14, wobei die Steuereinheit (12) an einem Ende der Rezirkulationsprozedur ausgestaltet ist zum:
a. Bewegen eines Abstellelements (70), das auf die Infusionsleitung (63) einwirkt, in eine Sperrposition, die Fluidfluss in einem zweiten Trakt (68) zulässt, der sich von der Verzweigungsstelle (67) bis zu dem zweiten Ende (63b) der Infusionsleitung (63) erstreckt und einen Fluidfluss in einem dritten Trakt (69) blockiert, der sich von der Verzweigungsstelle (67) zu dem dritten Ende (63c) der Infusionsleitung (63) erstreckt, um Fluidkommunikation zwischen der ersten Stelle (31) und der zweiten Stelle (32) durch den Rezirkulationsteil (66) hindurch zu verhindern;
b. Spülen des Blutes in dem zweiten Trakt (68) mit einem Fluid aus der Infusionsleitung (63).

## Revendications

1. Ensemble jetable (100) pour appareil de traitement extracorporel du sang (1) comprenant :
- une unité de filtration (2) ayant une entrée de sang (2a) et une sortie de sang (2b) ;
- un circuit de sang (17) comportant :
➢ une ligne de prélèvement de sang (6) s'étendant entre une première extrémité (6a) reliée à l'entrée de sang (2a) de l'unité de filtration (2) et une deuxième extrémité (6b) pour une liaison directe ou indirecte avec un patient (P) ;
➢ une ligne de retour de sang (7) s'étendant entre une première extrémité (7a) reliée à la sortie de sang (2b) de l'unité de filtration (2) et une deuxième extrémité (7b) pour une liaison directe ou indirecte avec le patient ;
> un conduit de pompe à sang (6p) situé sur le circuit de sang (17) et conçu pour coopérer avec une pompe à sang (21) de l'appareil de traitement extracorporel du sang pour favoriser la circulation sanguine dans le circuit de sang,
- une ligne de perfusion (63) s'étendant d'une première extrémité (63a) de raccordement à une source de fluide de perfusion (64),
- une ligne de perfusion avant la pompe à sang (51) s'étendant depuis une première extrémité (51a) de raccordement à une seconde source de fluide (10), et une deuxième extrémité (51b) fluidiquement reliée à la ligne de prélèvement de sang (6) entre la seconde extrémité (6b) de la ligne de prélèvement de sang (6) et le conduit de pompe à sang (6p),
dans lequel la ligne de perfusion (63) se ramifie au niveau d'un site de ramification (67) vers :
> une deuxième extrémité (63b) de la ligne de perfusion (63) ;
> une troisième extrémité (63c) de la ligne de perfusion (63) en communication fluidique directe ou indirecte avec la ligne de retour de sang (7) au niveau d'un second site (32), le second site (32) étant interposé entre le conduit de pompe à sang (6p) et la deuxième extrémité de la ligne de retour de sang (7) ;
**caractérisé en ce que** la deuxième extrémité (63b) de la ligne de perfusion (63) est directement reliée à la ligne de perfusion avant la pompe à sang (51) et en communication fluidique avec la ligne de prélèvement de sang (6) au niveau du premier site (31), le premier site (31) étant interposé entre la deuxième extrémité (6b) de la ligne de prélèvement de sang (6) et le conduit de pompe à sang (6p),
dans lequel la ligne de perfusion (63) et la ligne de perfusion avant la pompe à sang (51) sont reliées ensemble et présentent un conduit de perfusion commun s'étendant jusqu'au premier site (31) de la ligne de prélèvement de sang (6), dans lequel le circuit de sang est conçu pour permettre un écoulement de fluide dans un circuit de recirculation en boucle (11) comportant :
- une partie de la ligne de prélèvement de sang (6) interposée entre le premier site (31) de la ligne de prélèvement de sang (6) et l'entrée de sang (2a) de l'unité de filtration (2) ;
- le conduit de pompe à sang (6p) ;
- l'unité de filtration (2) ;
- une partie de la ligne de retour de sang (7) interposée entre la sortie de sang (2b) de l'unité de filtration (2) et le second site (32) de la ligne de retour de sang (7) ;
- une partie de recirculation (66) de la ligne de perfusion (63) en aval du site de ramification (67) et interposée entre le premier site (31) et le second site (32) ;
- le conduit de perfusion commun ;
le circuit de recirculation en boucle (11) étant configuré pour permettre au sang ou à un fluide, au moins dans un état de recirculation, de s'écouler en continu dans une circulation en boucle.

2. Ensemble jetable selon la revendication 1 précédente, dans lequel le circuit de recirculation en boucle (11) est configuré pour permettre une recirculation de fluide dans un premier sens du premier site (31) vers l'unité de filtration (2) et de l'unité de filtration (2) vers le second site (32) ou dans un second sens du second site (32) vers l'unité de filtration (2) et de l'unité de filtration (2) vers le premier site (31), et dans lequel :
- l'entrée de sang (2a) de l'unité de filtration (2) est en communication fluidique bidirectionnelle avec la partie de recirculation (66) de la ligne de perfusion (63) par le biais du premier site (31) ; et
- la sortie de sang (2b) de l'unité de filtration (2) est en communication fluidique bidirectionnelle avec la partie de recirculation (66) de la ligne de perfusion (63) par le biais du second site (32).

3. Ensemble jetable selon l'une quelconque des revendications 1 et 2 précédentes, dans lequel
le premier site (31) comprend un raccord de fluide, en particulier un raccord de fluide à trois voies, incluant :
- une entrée de sang reliée fluidiquement à la ligne de prélèvement (6) et en regard de la deuxième extrémité (6b) de la ligne de prélèvement de sang (6) ;
- une sortie de sang reliée fluidiquement à la ligne de prélèvement (6) et en regard de la première extrémité (6a) de la ligne de prélèvement de sang (6), éventuellement en regard du conduit pompe (6p) de la ligne de prélèvement de sang (6) ;
- une entrée de perfusion fluidiquement reliée à la partie de recirculation (66) de la ligne de perfusion (63).

4. Ensemble jetable selon l'une quelconque des revendications précédentes 1 à 3, comprenant en outre une unité jetable de réchauffeur de sang comprenant un trajet d'écoulement de sang doté d'une entrée et d'une sortie, l'unité jetable de réchauffeur de sang étant reliée fluidiquement au circuit de sang (17) en amont du second site (32), en particulier, l'entrée de l'unité jetable de réchauffeur de sang étant reliée fluidiquement à la ligne de retour de sang (7) et la sortie de l'unité jetable de réchauffeur de sang étant reliée fluidiquement à la ligne de retour de sang (7) en amont du second site (32).

5. Ensemble jetable selon l'une quelconque des revendications 1 à 4 précédentes, dans lequel la ligne de perfusion (63) s'étend depuis la première extrémité (63a) de la ligne de perfusion (63) pour un premier conduit (72), ledit premier conduit (72) se ramifiant au niveau du site de ramification (67) en :
- un deuxième conduit (68) s'étendant du site de ramification (67) jusqu'à la deuxième extrémité (63b) de la ligne de perfusion (63) ; et
- un troisième conduit (69) s'étendant de la ramification (67) jusqu'à la troisième extrémité (63c) de la ligne de perfusion (63),
la partie de recirculation (66) de la ligne de perfusion (63) comprenant le deuxième conduit (68) et le troisième conduit (69) de la ligne de perfusion (63),
dans lequel la ligne de perfusion (63) comprend un conduit de pompe de perfusion (65p) respectif disposé sur le premier conduit (72) de la ligne de perfusion (63) et configuré pour coopérer avec une pompe de perfusion (65) de l'appareil de traitement extracorporel du sang, ledit conduit de pompe de perfusion (65p) étant interposé entre la première extrémité (63a) et le site de ramification (67) de la ligne de perfusion (63).

6. Ensemble jetable selon l'une quelconque des revendications 1 à 5 précédentes, dans lequel la troisième extrémité (63c) de la ligne de perfusion (63) est reliée directement à la ligne de retour de sang (7) au niveau du second site (32) ou dans lequel l'ensemble jetable comprend un piège à bulles (19) conçu pour éliminer les bulles du sang pendant le traitement extracorporel du sang et disposé sur la ligne de retour (7) du circuit de sang (17), le second site (32) étant disposé au niveau du piège à bulles (19), et la troisième extrémité (63c) de la ligne de perfusion (63) étant reliée fluidiquement au piège à bulles (19), et dans lequel le circuit de recirculation en boucle (11) comprend en outre ledit piège à bulles (19).

7. Ensemble jetable selon la revendication 6 précédente, dans lequel le piège à bulles (19) comprend :
- une entrée de sang reliée à la partie de réception de la ligne de retour de sang (7), la partie de réception de la ligne de retour (7) étant interposée entre la sortie (2b) de l'unité de filtration (2) et ladite entrée de sang du piège à bulles (19) ;
- une sortie de sang reliée à un conduit secondaire de la ligne de retour de sang (7), le conduit secondaire de la ligne de retour de sang (7) étant interposé entre ladite sortie de sang du piège à bulles (19) et la deuxième extrémité (7b) de la ligne de retour (7) ;
- une liaison auxiliaire reliée à la troisième extrémité (63c) de la ligne de perfusion (63).

8. Ensemble jetable selon l'une quelconque des revendications 1 à 7 précédentes, dans lequel la ligne de perfusion avant la pompe à sang (PBP) (51) comprend un conduit de pompe à perfusion PBP (51p) respectif configuré pour coopérer avec une pompe à perfusion PBP (54),
dans lequel la deuxième extrémité (63b) de la ligne de perfusion (63) est directement reliée à la ligne de perfusion avant la pompe à sang (51) en aval du conduit de pompe de perfusion PBP (51p).

9. Ensemble jetable selon l'une quelconque des revendications 1 à 8 précédentes, comprenant en outre un site de détection de pression (52a) agencé sur la ligne de perfusion avant la pompe à sang (51) entre un conduit de pompe de perfusion PBP (51p) de la ligne de perfusion avant la pompe à sang (51) et la deuxième extrémité (51b) de la ligne de perfusion avant la pompe à sang (51), dans lequel le site de détection de pression (52a) est agencé entre le conduit de pompe à perfusion PBP (51p) et la deuxième extrémité (63b) de la ligne de perfusion (63), en particulier ledit site de détection de pression (52a) étant une capsule de pression.

10. Ensemble jetable selon l'une quelconque des revendications 1 à 9 précédentes, dans lequel l'unité de filtration (2) comprend :
> une chambre primaire (3) reliée fluidiquement au circuit de sang par le biais de l'entrée de sang et de la sortie de sang de l'unité de filtration (2),
➢ une chambre secondaire (4) reliée fluidiquement ou pouvant l'être à un circuit de fluide (16), ladite chambre secondaire (4) étant conçue pour recevoir un fluide de dialyse et/ou pour éliminer un fluide effluent, et dans lequel la chambre primaire (3) est séparée de la chambre secondaire (4) par une membrane semi-perméable (5),
l'ensemble jetable comprenant en outre un circuit de fluide (16) comportant :
- une ligne de fluide effluent (13) s'étendant entre une première extrémité de raccordement à une unité d'évacuation, telle qu'un récipient de collecte (62), et une deuxième extrémité reliée fluidiquement à une sortie de fluide d'une chambre secondaire (4) de l'unité de filtration (2), dans lequel ladite ligne de fluide effluent (13) comprend un conduit de pompe d'effluent (13p) conçu pour coopérer avec une pompe d'effluent (26) de l'appareil de traitement extracorporel du sang pour favoriser l'écoulement de fluide effluent depuis la sortie de fluide de la chambre secondaire (4) vers l'unité d'évacuation ;
- éventuellement une ligne d'alimentation en fluide (8) s'étendant entre une première extrémité de raccordement à une source de fluide de dialyse (14), et une deuxième extrémité reliée fluidiquement à une entrée de fluide d'une chambre secondaire (4) de l'unité de filtration (2), dans lequel ladite ligne d'alimentation en fluide (8) comprend un conduit de pompe d'alimentation (8p) configuré pour coopérer avec une pompe d'alimentation (25) de l'appareil extracorporel de traitement du sang pour faire progresser le fluide de dialyse vers l'entrée de fluide de la chambre secondaire (4) de l'unité de filtration (2).

11. Appareil de traitement extracorporel du sang comportant :
- une ou plusieurs pompes (21, 25, 26, 54, 65, 75) comprenant une pompe à sang (21),
- un ou plusieurs éléments d'interception (20, 27, 59, 70),
- un ensemble jetable (100) selon l'une quelconque des revendications précédentes
- une unité de commande (12) configurée pour commander lesdites une ou plusieurs pompes (21, 25, 26, 54, 65, 75) et lesdits un ou plusieurs éléments d'interception (20, 27, 59, 70) dans le but de définir différents circuits d'écoulement à l'intérieur du circuit de sang (17) et de la ligne de perfusion (63), l'unité de commande (12) étant configurée pour définir au moins les circuits d'écoulement suivants :
> un circuit d'écoulement de traitement, dans lequel les une ou plusieurs pompes (21, 25, 26, 54, 65, 75) et les un ou plusieurs éléments d'interception (20, 27, 59, 70) sont conçus pour permettre au sang de s'écouler de la deuxième extrémité (6b) de la ligne de prélèvement de sang (6) à l'unité de filtration (2) et de l'unité de filtration (2) à la deuxième extrémité (7b) de la ligne de retour de sang (7) ; et
> un circuit de recirculation en boucle (11), dans lequel les une ou plusieurs pompes (21, 25, 26, 54, 65, 75) et les un ou plusieurs éléments d'interception (20, 27, 59, 70) sont conçus pour permettre au sang ou à un fluide de s'écouler en continu dans une circulation en boucle,
dans lequel la ligne de perfusion (63) et la ligne de perfusion avant la pompe à sang (51) sont reliées ensemble et présentent un conduit de perfusion commun s'étendant jusqu'au premier site (31) de la ligne de prélèvement de sang (6), dans lequel le circuit de recirculation en boucle (11) comporte :
• une partie de la ligne de prélèvement de sang (6) interposée entre le premier site (31) de la ligne de prélèvement de sang (6) et l'entrée de sang (2a) de l'unité de filtration (2) ;
• le conduit de pompe à sang (6p) ;
• l'unité de filtration (2) ;
• une partie de la ligne de retour de sang (7) interposée entre la sortie de sang (2b) de l'unité de filtration (2) et le second site (32) de la ligne de retour de sang (7) ;
• une partie de recirculation (66) de la ligne de perfusion (63) en aval du site de ramification (67) et interposée entre le premier site (31) et le second site (32) ;
• le conduit de perfusion commun.

12. Appareil selon la revendication 11 précédente, dans lequel l'unité de commande (12) est configurée pour réaliser une procédure de recirculation comprenant les étapes de :
- définition du circuit de recirculation en boucle (11) au moins en :
> commandant les un ou plusieurs éléments d'interception (20, 27, 59, 70) pour mettre en communication fluidique le premier site (31) avec le second site (32) par le biais de la partie de recirculation (66) de la ligne de perfusion (63) ;
- commandant la pompe à sang (21) pour déterminer un écoulement de sang à l'intérieur du circuit de recirculation en boucle (11) du circuit de sang (17) de l'ensemble jetable,
en particulier dans lequel les un ou plusieurs éléments d'interception (20, 27, 59, 70) comprennent un élément d'interception (70) agissant sur la ligne de perfusion (63) et mobile entre :
> une position de recirculation, dans laquelle l'élément d'interception (70) est conçu pour permettre une communication de fluide entre le premier site (31) et le second site (32) par le biais de la partie de recirculation (63) de la ligne de perfusion (63) ;
> une position de verrouillage, dans laquelle l'élément d'interception (70) est conçu pour empêcher la communication de fluide entre le premier site (31) et le second site (32) par le biais de la partie de recirculation (63) de la ligne de perfusion (63) ;
et dans lequel, dans la procédure de recirculation, l'unité de commande (12) est configurée pour basculer l'élément d'interception (70) en position de recirculation.

13. Appareil selon la revendication 12, dans lequel, avant de débuter la procédure de recirculation, l'unité de commande (12) est configurée pour :
a. arrêter la pompe à sang (21) ;
b. avant de basculer l'élément d'interception (70) en position de recirculation, agir sur le circuit de sang (17) pour réduire une différence de pression entre des parties du circuit de sang (17) au travers de la pompe à sang (21) ;
en particulier dans lequel pour réduire la différence de pression, l'unité de commande (12) est configurée pour réaliser au moins l'une des actions suivantes :
i. commander les un ou plusieurs éléments d'interception (20, 27, 59, 70) d'une position fermée à une position ouverte pour basculer entre l'empêchement et l'autorisation du passage de fluide dans la ligne de prélèvement de sang (6) entre la deuxième extrémité (6b) de la ligne de prélèvement de sang (6) et le premier site (31) et/ou dans la ligne de retour de sang (7) entre le second site (32) et la deuxième extrémité (7b) de la ligne de retour de sang (7) ;
ii. perfuser un fluide de perfusion dans le circuit de sang (17) pour ajuster une pression d'accès et/ou une pression de retour de manière à les amener à une valeur plus proche, par exemple à une différence de pression inférieure à 100 mmHg.

14. Appareil selon l'une quelconque des revendications 11 à 13 précédentes, dans lequel une ou plusieurs pompes (21, 25, 26, 54, 65, 75) comportent une pompe à perfusion (65) active sur le conduit de pompe à perfusion (63p) de la ligne de perfusion,
dans lequel, dans la procédure de recirculation, l'unité de commande (12) est configurée pour :
a. garder ladite pompe à perfusion (65) inactive, en particulier la pompe à perfusion (65) étant une pompe occlusive, telle qu'une pompe péristaltique ;
b. arrêter toute ultrafiltration de fluides d'une chambre primaire (3) vers une chambre secondaire (4) de l'unité de filtration (2) ;
c. recirculer le sang dans le circuit de recirculation en boucle (11) pour empêcher le sang de coaguler ;
d. facultativement, faire fonctionner la pompe à sang (21) à une vitesse différente de celle de la pompe à sang (21) pendant le traitement extracorporel du sang, en particulier, pendant la procédure de recirculation, l'unité de commande (12) est configurée pour faire fonctionner la pompe à sang (21) dans la même direction que pendant le traitement extracorporel du sang.

15. Appareil selon l'une quelconque des revendications 11 à 14 précédentes, dans lequel, à la fin de la procédure de recirculation, l'unité de commande (12) est configurée pour :
a. déplacer un élément d'interception (70) agissant sur la ligne de perfusion (63) vers une position de verrouillage permettant l'écoulement du fluide dans un deuxième conduit (68) s'étendant du site de ramification (67) jusqu'à la deuxième extrémité (63b) de la ligne de perfusion (63) et bloquant un écoulement de fluide dans un troisième conduit (69) s'étendant du site de ramification (67) jusqu'à la troisième extrémité (63c) de la ligne de perfusion (63) pour empêcher une communication fluidique entre le premier site (31) et le second site (32) par le biais de la partie de recirculation (66) ;
b. rincer le sang dans le deuxième conduit (68) avec un fluide provenant de la ligne de perfusion (63).
